(19) Europäisches Patentamt
European Patent Office
Office européen des brevets

(11) EP 0 931 077 B1

(12) EUROPEAN PATENT SPECIFICATION

(45) Date of publication and mention of the grant of the patent: **27.04.2005 Bulletin 2005/17**

(21) Application number: **97941476.0**

(22) Date of filing: **11.09.1997**

(51) Int Cl.[7]: **C07D 401/14**, A61K 31/435, C07D 401/04

(86) International application number: **PCT/US1997/015902**

(87) International publication number: **WO 1998/011097 (19.03.1998 Gazette 1998/11)**

(54) **SUBSTITUTED BENZOCYCLOHEPTAPYRIDINE USEFUL AS INHIBITORS OF FARNESYL-PROTEIN TRANSFERASE**

SUBSTITUIERTE BENZOCYCLOHEPTAPYRIDINE DERIVATE VERWENDBAR ALS FARNESYL-PROTEIN TRANSFERASE INHIBITOREN

BENZOCYCLOHEPTAPYRIDINE SUBSTITUEE UTILE EN TANT QU'INHIBITEUR DE LA FARNESYL-PROTEINE TRANSFERASE

(84) Designated Contracting States:
**AT BE CH DE DK ES FI FR GB GR IE IT LI LU NL PT SE**
Designated Extension States:
**LT LV RO**

(30) Priority: **13.09.1996 US 713705**

(43) Date of publication of application: **28.07.1999 Bulletin 1999/30**

(73) Proprietor: **SCHERING CORPORATION**
**Kenilworth New Jersey 07033 (US)**

(72) Inventors:
- **RANE, Dinanath, F.**
  **Morganville, NJ 07751 (US)**
- **COOPER, Alan, B.**
  **West Caldwell, NJ 07006 (US)**
- **MALLAMS, Alan, K.**
  **Hackettstown, NJ 07840 (US)**
- **DOLL, Ronald, J.**
  **Maplewood, NJ 07040 (US)**
- **NJOROGE, F., George**
  **Union, NJ 07083 (US)**
- **TAVERAS, Arthur, G.**
  **Rockaway, NJ 07866 (US)**

(74) Representative: **Ritter, Stephen David et al**
**Mathys & Squire**
**120 Holborn**
**London EC1N 2SQ (GB)**

(56) References cited:
**EP-A- 0 396 083**
**WO-A-92/00293**
**WO-A-95/10515**
**WO-A-95/10516**
**GB-A- 1 593 417**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 931 077 B1

## Description

This invention relates to substituted benzocycloheptapyridines useful as inhibitors of farnesyl-protein transferase.

## BACKGROUND

Substituted benzo[5,6]cycloheptapyridines and 1,5,6,11-tetrahydrobenzo [5,6] cyclohepta [1,2-b]pyrazolo [4,3-e]pyridine derivatives having anti-inflammatory activity and anti-allergic activity are described in GB-A-1593417, EP-A-0396083 and WO-A-92/00293.

Substituted benzo[5,6]cycloheptapyridines having Ras function inhibitory activity are described in WO-A-95/10515 and WO-A-95/10516.

Patent application WO 95/00497 published 5 January 1995 under the Patent Cooperation Treaty (PCT) describes compounds which inhibit the enzyme, famesyl-protein transferase (FTase) and the famesylation of the oncogene protein Ras. Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer.

To acquire transforming potential, the precursor of the Ras oncoprotein must undergo famesylation of the cysteine residue located in a carboxyl-terminal tetrapeptide. Inhibitors of the enzyme that catalyzes this modification, famesyl protein transferase, have therefore been suggested as anticancer agents for tumors in which Ras contributes to transformation. Mutated, oncogenic forms of Ras are frequently found in many human cancers, most notably in more than 50% of colon and pancreatic carcinomas (Kohl et al., Science, Vol. 260, 1834 to 1837, 1993).

In view of the current interest in inhibitors of famesyl protein . transferase, a welcome contribution to the art would be additional compounds useful for the inhibition of famesyl protein transferase. Such a contribution is provided by this invention.

## SUMMARY OF THE INVENTION

Inhibition of famesyl protein transferase by tricyclic compounds of this invention has not been reported previously. Thus, this invention provides a method for inhibiting famesyl protein transferase using tricyclic compounds of this invention which: (i) potently inhibit famesyl protein transferase, but not geranylgeranyl protein transferase I, in vitro; (ii) block the phenotypic change induced by a form of transforming Ras which is a famesyl acceptor but not by a form of transforming Ras engineered to be a geranylgeranyl acceptor; (iii) block intracellular processing of Ras which is a famesyl acceptor but not of Ras engineered to be a geranylgeranyl acceptor; and (iv) block abnormal cell growth in culture induced by transforming Ras. Several compounds of this invention have been demonstrated to have anti-tumor activity in animal models.

This invention provides a method for inhibiting the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

Compounds useful in the claimed methods are represented by Formula 1.0:

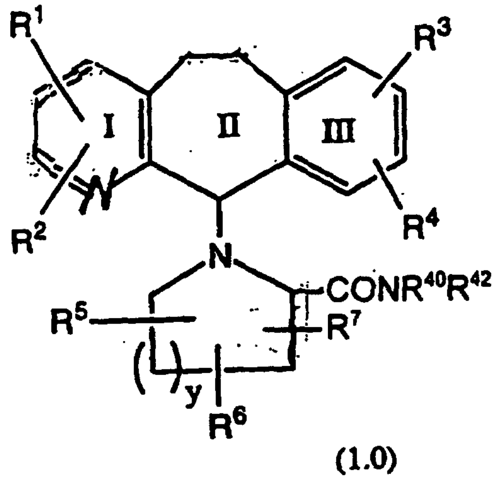

(1.0)

or a pharmaceutically acceptable salt or solvate thereof, wherein:

$R^1$ and $R^4$ are H and $R^2$ and $R^3$ are halo selected from chloro and bromo; or $R^1$ is H and $R^2$, $R^3$ and $R^4$ are halo selected from chloro and bromo.

$R^5$ and $R^6$ (y = 0) or $R^5$, $R^6$ and $R^7$ (y = 1) each independently represents H, $-CF_3$, $-COR^{10}$, alkyl or aryl, said alkyl or aryl optionally being substituted with $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$, $OPO_3R^{10}$ or one of $R^5$, $R^6$ and $R^7$ can be taken in combination with $R^{40}$ as defined below to represent $-(CH_2)_r-$ wherein r is 1 to 4 which can be substituted with $C_{1-6}$ alkyl, $C_{1-6}$ alkoxy, $-CF_3$ or aryl, or $R^5$ is combined with $R^6$ or $R^7$ to represent = O or = S;

$R^{10}$ independently represents H, alkyl, cycloalkyl, cycloalkylalkyl, heteroaryl, aryl, aralkyl or $-NR^{40}R^{42}$;

$R^{11}$ represents alkyl or aryl;

$R^{40}$ and $R^{42}$ independently represent H, aryl, alkyl, aralkyl, heteroaryl, heteroarylalkyl, heterocycloalkyl, heterocycloalkylalkyl, heteroalkyl, cycloalkyl, cycloalkylalkyl, alkenyl and alkynyl;

y is 0 (zero) or 1;

and wherein:

alkyl-(including the alkyl portions of alkoxy, alkylamino and dialkylamino)-represents straight and branched carbon chains and contains from one to twenty carbon atoms, wherein said alkyl group may be optionally and independently substituted with one, two, three or more optional substituents;

alkenyl-represents straight and branched carbon chains having at least one carbon to carbon double bond and containing from 2 to 12 carbon atoms, wherein said alkenyl group may be optionally and independently substituted with one, two, three or more optional substituents;

alkynyl-represents straight and branched carbon chains having at least one carbon to carbon triple bond and containing from 2 to 12 carbon atoms, wherein said alkynyl group may be optionally and independently substituted with one, two, three or more optional substituents;

aryl (including the aryl portion of aralkyl)-represents a carbocyclic group containing from 6 to 15 carbon atoms and having at least one aromatic ring, wherein said aryl group optionally can be fused with aryl, cycloalkyl, heteroaryl or heterocycloalkyl rings; and wherein any of the available substitutable carbon and nitrogen atoms in said aryl group and/or said fused ring(s) may be optionally and independently substituted with one, two, three or more optional substituents;

aralkyl - represents an alkyl group, as defined above, wherein one or more hydrogen atoms of the alkyl moiety have been substituted with one or more aryl groups; wherein said aralkyl group may be optionally and independently substituted with one, two, three or more optional substituents;

cycloalkyl - represents saturated carbocyclic rings branched or unbranched of from 3 to 20 carbon atoms, wherein said cycloalkyl group may be optionally and independently substituted with one, two, three or more optional substituents;

cycloalkylalkyl - represents an alkyl group, as defined above, wherein one or more hydrogen atoms of the alkyl moiety have been substituted with one or more cycloalkyl groups; wherein said cycloalkylalkyl group may be optionally and independently substituted with one, two, three or more optional substituents;

heteroaryl-represents cyclic groups having at least one heteroatom selected from O, S and N, said heteroatoms (s) interrupting a carbocyclic ring structure and having a sufficient number of delocalized pi electrons to provide aromatic character, with the aromatic heterocyclic groups containing from 2 to 14 carbon atoms, wherein said heteroaryl group optionally can be fused with one or more aryl, cycloalkyl, heteroaryl or heterocycloalkyl rings; and wherein any of the available substitutable carbon or nitrogen atoms in said heteroaryl group and/or said fused ring (s) may be optionally and independently substituted with one, two, three or more optional substituents;

heteroarylalkyl - represents an alkyl group, as defined above, wherein one or more hydrogen atoms have been replaced by one or more heteroaryl groups; wherein said heteroarylalkyl group may be optionally and independently substituted with one, two, three or more optional substituents;

heterocycloalkyl - represents a saturated, branched or unbranched carbocyclic ring containing from 3 to 15 carbon atoms, which carbocyclic ring is interrupted by 1 to 3 heteroatoms selected from -O-, -S- and -N-, wherein optionally, said ring may contain one or two unsaturated bonds which do not impart aromatic character to the ring; and wherein any of the available substitutable carbon and nitrogen atoms in the ring may be optionally and independently substituted with one, two, three or more optional substituents; and

"optional substituents" are selected from halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $SO_2R^{10}$, $-SOR^{10}$, $-SR^{10}$, $-NHSO_2$, $-NO_2$, $-CONR^{10}$, $-NCOR^{10}$ or $-COOR^{10}$.

In the compounds of Formula (1.0), preferably $R^2$ is Br and $R^3$ is Cl. These compounds include compounds wherein $R^2$ is in the 3-position and $R^3$ is in the 8-position, e.g., 3-Br and 8-Cl.

Also, compounds of Formula (1.0) preferably include compounds wherein $R^2$ is in the 3-position, $R^3$ is in the 7-position and $R^4$ is in the 8-position, e.g., 3-Br, 7-Br, 8-Cl. Also included are compounds wherein $R^2$ is in the 3-position, $R^3$ is in the 8-position and $R^4$ is in the 10-position, e.g. 3-Br, 8-Cl and 10-Br.

Preferably one of $R^{40}$ or $R^{42}$ is H. Also preferred is that $R^{40}$ is H and $R^{42}$ is 3-pyridylmethyl.

In another embodiment, the present invention is directed toward a pharmaceutical composition for inhibiting the abnormal growth of cells comprising an effective amount of compound (1.0) in combination with a pharmaceutically acceptable carrier.

In another embodiment, the present invention is directed toward compounds of formula (1.0) for inhibiting the abnormal growth of cells, including transformed cells, comprising administering an effective amount of compound (1.0) to a mammal (e.g., a human) in need of such treatment. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; (3) benign and malignant cells of other proliferative diseases in which aberrant Ras activation occurs, and (4) benign or malignant cells that are activated by mechanisms other than the Ras protein. Without wishing to be bound by theory, it is believed that these compounds may function either through the inhibition of G-protein function, such as ras p21, by blocking G-protein isoprenylation, thus . making them useful in the treatment of proliferative diseases such as tumor growth and cancer, or through inhibition of ras famesyl protein transferase, thus making them useful for their antiproliferative activity against ras transformed cells.

The cells to be inhibited can be tumor cells expressing an activated ras oncogene. For example, the types of cells that may be inhibited include pancreatic tumor cells, lung cancer cells, myeloid leukemia tumor cells, thyroid follicular tumor cells, myelodysplastic tumor cells, epidermal carcinoma tumor cells, bladder carcinoma tumor cells or colon tumors cells. Also, the inhibition of the abnormal growth of cells by the treatment with compound (1.0) may be by inhibiting ras famesyl protein transferase. The inhibition may be of tumor cells wherein the Ras protein is activated as a result of oncogenic mutation in genes other than the Ras gene. Alternatively, compounds (1.0) may inhibit tumor cells activated by a protein other than the Ras protein.

Compounds of formula (1.0) may be used for inhibiting tumor growth by administering an effective amount of compound (1.0) to a mammal (e.g., a human) in need of such treatment. This provides a method for inhibiting the growth of tumors expressing an activated Ras oncogene by the administration of an effective amount of the above described compounds. Examples of tumors which may be inhibited include, but are not limited to, lung cancer (e.g., lung adenocarcinoma), pancreatic cancers (e.g., pancreatic carcinoma such as, for example, exocrine pancreatic carcinoma), colon cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS), bladder carcinoma and epidermal carcinoma.

It is believed that compounds of this invention inhibit proliferative diseases, both benign and malignant, wherein Ras proteins are aberrantly activated as a result of oncogenic mutation in other genes--i.e., the Ras gene itself is not activated by mutation to an oncogenic form--with said inhibition being accomplished by the administration of an effective amount of the carbonyl piperazinyl and piperidinyl compounds (1.0) described herein, to a mammal (e.g., a human) in need of such treatment. For example, the benign proliferative disorder neurofibromatosis, or tumors in which

Ras is activated due to mutation or overexpression of tyrosine kinase oncogenes (e.g., neu, src, abl, lck, and fyn), may be inhibited by the carbonyl piperazinyl and piperidinyl compounds (1.0) described herein.

In another embodiment, the compounds of formula (1.0) may be used for inhibiting ras famesyl protein transferase and the famesylation of the oncogene protein Ras by administering an effective amount of compound (1.0) to marrimals, especially humans. The administration of the compounds of this invention to patients, to inhibit famesyl protein transferase, is useful in the treatment of the cancers described above.

DETAILED DESCRIPTION OF THE INVENTION

The following solvents and reagents are referred to herein by the abbreviations indicated:

tetrahydrofuran (THF);
ethanol (EtOH);
methanol (MeOH);
ethyl acetate (EtOAc);
N,N-dimethylformamide (DMF);
trifluoroacetic acid (TFA);
1-hydroxybenzotriazole (HOST);
1-(3-dimethylaminopropyl)-3-ethyl carbodiimide hydrochloride (DEC);
dimethylsulfoxide (DMSO);
acetic acid (HOAc or AcOH)
4-methylmopholine (NMM);
dimethylaminopyridine (DMAP); and
dimethoxyethane (DME).
t-butoxycarbonyl (BOC)
acetyl(OAc)

As used herein, the following terms are used as defined below unless otherwise indicated:

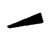 or 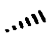 - indicates a pure isomer;
 - when attached to a carbon atom labeled with an asterisk (*), indicates a separated isomer whose stereochemistry is not established;
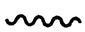 - indicates a racemic mixture;
$M^+$ -represents the molecular ion of the molecule in the mass spectrum;
$MH^+$ -represents the molecular ion plus hydrogen of the molecule in the mass spectrum;
PMR or NMR refers to proton magnetic resonance spectroscopy or nuclear magnetic resonance spectroscopy, whose terms are interchangeable;
Bu-represents butyl;
Et-represents ethyl;
Me-represents methyl;
Ph-represents phenyl;
benzotriazol-1-yloxy represents

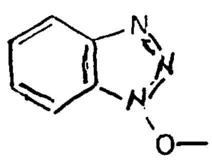 ;

1-methyl-tetrazoi-5-ylthio represents

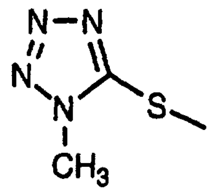

;

acyl-a moiety of the formula $-COR^{15}$ wherein $R^{15}$ represents H, $C_{1-6}$ alkyl, aryl, aralkyl, heteroalkyl, heteroaryl, heteroarylalkyl, heterocycloalkyl and heterocycloalkylalkyl;

alkyl-(including the alkyl portions of alkoxy, alkylamino and dialkylamino)-represents straight and branched carbon chains and contains from one to twenty carbon atoms, preferably one to six carbon atoms (i.e. $C_{1-6}$ alkyl); for example methyl, ethyl, propyl, iso-propyl, n-butyl, t-butyl, n-pentyl, isopentyl, hexyl and the like; wherein said alkyl and said $C_{1-6}$ alkyl group may be optionally and independently substituted with one, two, three or more of the following: halo, alkyl, aryl, alkoxy, amino ($-NH_2$), alkylamino, cyano (-CN), $-CF_3$, dialkylamino, hydroxy, oxy (=O), phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-NCOR^{10}$ or $-COOR^{10}$.

alkoxy-an alkyl moiety of one to 20 carbon atoms covalently bonded to an adjacent structural element through an oxygen atom, for example, methoxy, ethoxy, propoxy, butoxy, pentoxy, hexoxy and the like; wherein said alkoxy group may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$;

alkoxycarbonyl - represents a alkoxy moiety, as defined above, covalantly bonded to a carbonyl moiety (-CO-) through an oxygen atom, for example, $-COOCH_3$, $-COOCH_2CH_3$ and $-COOC(CH_3)_3$;

alkenyl-represents straight and branched carbon chains having at least one carbon to carbon double bond and containing from 2 to 12 carbon atoms, preferably from 2 to 6 carbon atoms and most preferably from 3 to 6 carbon atoms wherein said alkenyl group may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$;

alkynyl-represents straight and branched carbon chains having at least one carbon to carbon triple bond and containing from 2 to 12 carbon atoms, preferably from 2 to 6 carbon atoms; wherein said alkynyl group may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$;

amino acid- refers to organic compounds having both an amino group ($-NH_2$) and a carboxyl group (-COOH). Representative amino acids include glycine, serine, alanine, phenylalanine, tyrosine, S-methyl methionine and histidine;

aryl (including the aryl portion of aralkyl)-represents a carbocyclic group containing from 6 to 15 carbon atoms and having at least one aromatic ring (e.g., aryl is phenyl), wherein said aryl group optionally can be fused with aryl, cycloalkyl, heteroaryl or heterocycloalkyl rings; and wherein any of the available substitutable carbon and nitrogen atoms in said aryl group and/or said fused ring(s) may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$;

aralkyl - represents an alkyl group, as defined above, wherein one or more hydrogen atoms of the alkyl moiety have been substituted with one or more aryl groups; wherein said aralkyl group may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$; Representative aralkyl groups include benzyl and diphenylmethyl;

aralkyloxy - represents an aralkyl group, as defined above. covalently bonded to an adjacent structural element through an oxygen atom, for example, phenylmethyloxy and phenylethyloxy;

aralkyloxycarbonyl - represents an aralkyloxy group, as defined above, covalantly bonded to a carbonyl moiety (-CO-) through an oxygen atom, for example, $-COOCH_2C_6H_5$ and $-COOCH_2CH_2C_6H_5$;

carboxamido - represents a moiety of the formula $-CONH_2$ or $-CONR^{40}R^{42}$;

cycloalkyl-represents saturated carbocyclic rings branched or unbranched of from 3 to 20 carbon atoms, preferably 3 to 7 carbon atoms; wherein said cycloalkyl group may be optionally and independently substituted with one, two,

three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$;

cycloalkylalkyl - represents an alkyl group, as defined above, wherein one or more hydrogen atoms of the alkyl moiety have been substituted with one or more cycloalkyl groups; wherein said cycloalkylalkyl group may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$;

halo-represents fluoro, chloro, bromo and iodo;

heteroalkyl-represents straight and branched carbon chains containing from one to twenty carbon atoms, preferably one to six carbon atoms interrupted by 1 to 3 heteroatoms selected from -O-, -S- and -N-; wherein any of the available substitutable carbon and nitrogen atoms in said heteroalkyl chain may be optionally and independendently substituted with one, two, three or more of the following: halo, $C_1$-$C_6$ alkyl, aryl, cyano, hydroxy, alkoxy, oxy, phenoxy, $-CF_3$, $-OCF_3$, amino, alkylamino, dialkylamino, heterocycloalkyl, $-SO_2NH_2$, $-NHSO_2R^{10}$, $-SO_2NHR^{10}$, $-SO_2R^{10}$, $-SOR^{10}$, $-SR^{10}$, or $-NHSO_2$, $-NO_2$, $-CONR^{10}$, $-NCOR^{10}$ or $-COOR^{10}$;

heteroaryl-represents cyclic groups having at least one heteroatom selected from O, S and N, said heteroatom(s) interrupting a carbocyclic ring structure and having a sufficient number of delocalized pi electrons to provide aromatic character with the aromatic heterocyclic groups. containing from 2 to 14 carbon atoms,wherein said heteroaryl group optionally can be fused with one or more aryl, cycloalkyl, heteroaryl or heterocycloalkyl rings; and wherein any of the available substitutable carbon or nitrogen atoms in said heteroaryl group and/or said fused ring (s) may be optionally and independendently substituted with one, two, three or more of the following: halo, $C_1$-$C_6$ alkyl, aryl, cyano, hydroxy, alkoxy, oxy, phenoxy, $-CF_3$, $-OCF_3$, amino, alkylamino, dialkylamino, heterocycloalkyl, $-SO_2NH_2$, $-NHSO_2R^{10}$, $-SO_2NHR^{10}$, $-SO_2R^{10}$, $-SOR^{10}$, $-SR^{10}$, or $-NHSO_2$, $-NO_2$, $-CONR^{10}$, $-NCOR^{10}$ or $-COOR^{10}$. Representative heteroaryl groups can include, for example, furanyl, imidazoyl, pyrimidinyl, triazolyl, 2-, 3- or 4-pyridyl or 2-, 3- or 4-pyridyl N-oxide wherein pyridyl N-oxide can be represented as:

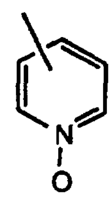
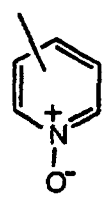
or
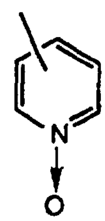
,

heteroarylalkyl - represents an alkyl group, as defined above, wherein one or more hydrogen atoms have been replaced by one or more heteroaryl groups; wherein said heteroarylalkyl group may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$; as exemplified by 2-, 3- or 4-pyridylmethyl or 2-, 3- or 4-pyridylmethyl N-oxide;

heterocycloalkyl-represents a saturated, branched or unbranched carbocylic ring containing from 3 to 15 carbon atoms, preferably from 4 to 6 carbon atoms, which carbocyclic ring is interrupted by 1 to 3 heteroatoms selected from -O-, -S- and -N- , wherein optionally, said ring may contain one or two unsaturated bonds which do not impart aromatic character to the ring; and wherein any of the available substitutable carbon and nitrogen atoms in the ring may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NO_2$, $-CONR^{10}$, $-NCOR^{10}$ or $-COOR^{10}$

Representative heterocycloalkyl groups can include 2- or 3-tetrahydrofuranyl, 2- or 3- tetrahydrothienyl, 1-, 2-, 3- or 4-piperidinyl, 2- or

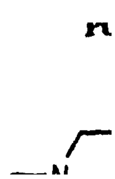

3-pyrrolidinyl, 1-, 2- or 3-piperizinyl, 2- or 4-dioxanyl, wherein t is 0, 1 or 2; morpholinyl,heterocycloalkylalkyl- represents an alkyl group, as defined above, wherein one or more hydrogen atoms have been replaced by one or more heterocycloalkyl groups; wherein optionally, said ring may contain one or two unsaturated bonds which do not impart aromatic character to the ring; and wherein said heterocycloalkylalkyl group may be optionally and independently substituted with one, two, three or more of the following: halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $-SO_2R^{10}$, $-SOR^{10}$, $-SR^{10}$, $-NHSO_2$, $-NO_2$, $-CONR^{10}$, $-NCOR^{10}$ or $-COOR^{10}$;

imido - represents a moiety of the formula

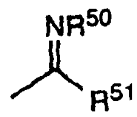

wherein and $R^{50}$ represents H, cyano, aryl, $-SO_2NH_2$, $-SO_2NR^{40}R^{42}$ and carboxamido and $R^{51}$ represents aryl and aryloxy.

Reoresentative imido groups can include, for example,

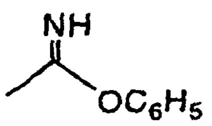 , 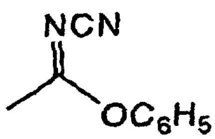 , 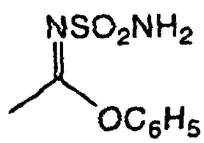 , 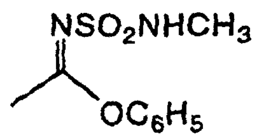 ,

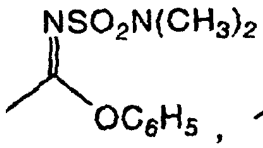 , 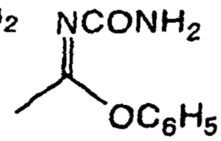 , 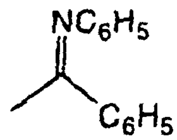 , 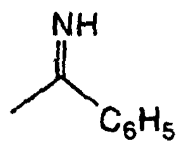

imidamido - represents a moiety of the formula

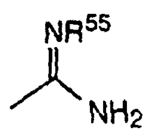

wherein and $R^{55}$ represents H, cyano, $-SO_2NH_2$, $-SO_2NR^{40}R^{42}$, carboxamido, hydroxy and alkoxy. Representative imidamido groups can include, for example,

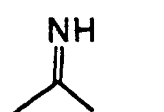 , 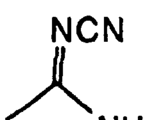 , 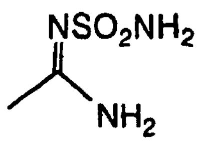 , 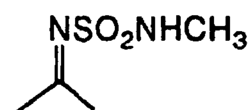 , 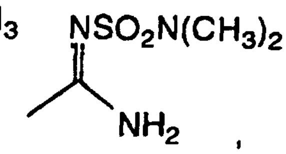 ,

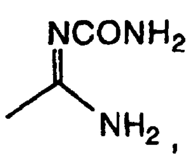 , 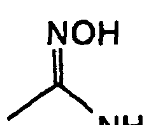 , and 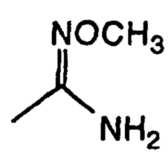 ;

N-glycosyl- represents a pyranosyl or furanosyl monosaccharide. Representative N-glycosyl groups include (N → 1)-tetra-O-acetyl-D-glucosyl, (N → 1 )-tetra-O-acetyl-D-galactosyl and (N → 1) -tri-O-acetyl-D-ribosyl, e.g.

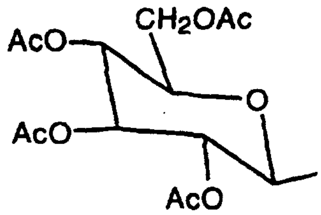
,
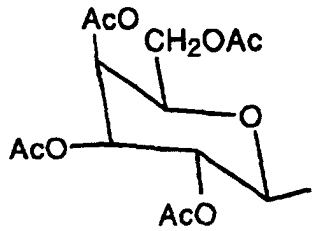
and
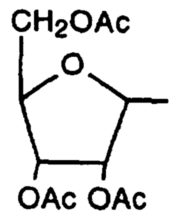
;

D-glycosyl D-galactosyl D-ribosyl

1-amino-2-nitroethenyl represents the formula:

$$-C(NHCH_3)=CHNO_2;$$

dialkylphosphinyl - represents a phosphine (-PO) moiety covalently bonded to two alkyl groups. A representative dialkylphosphinyl group is $-PO(CH_3)_2$.

sulfamoyl - represents a moiety of the formula $-SO_2R^{60}$ wherein $R^{60}$ represents amino, alkylamino and dialkylamino. Representative sulfamoyl groups can include, for example, $-SO_2NH_2$, $-SO_2NHCH_3$, $-SO_2N(CH_3)_2$.

sulfonyl - represents a moiety of the formula $-SO_2R^{60}$ wherein $R^{60}$ represents alkyl, aryl and arylalkyl. Representative sulfonyl groups can include, for example, $-SO_2CH_3$, $-SO_2C_6H_5$, $-SO_2C_6H_4CH_3$, and $-SO_2CH_2C_6H_5$.

Reference to the position of the substituents $R^1$, $R^2$, R3, and R4 is based on the numbered ring structure:

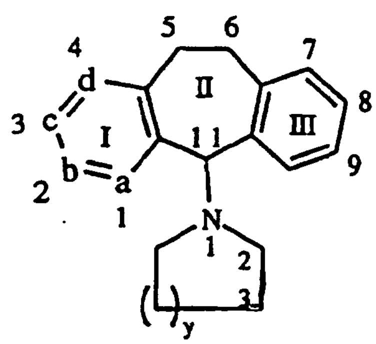

Certain compounds of the invention may exist in different stereoisomeric forms (e.g., enantiomers and diastereoisomers). The invention contemplates all such stereoisomers both in pure form and in mixture, including racemic mixtures. For example, the carbon atom at the C-11 position can be in the S or R stereoconfiguration. Also, the carbon atom at the C-2 and C-3 positions of the pyrrolidine (y=1) or at the C-2 position of the azetidine moiety (y=0) bonded at C-11 can also be in the S or R stereoconfiguration.

Certain tricyclic compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous

NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

Compounds of the present invention can be prepared according to the following Scheme 1:

**Scheme 1**

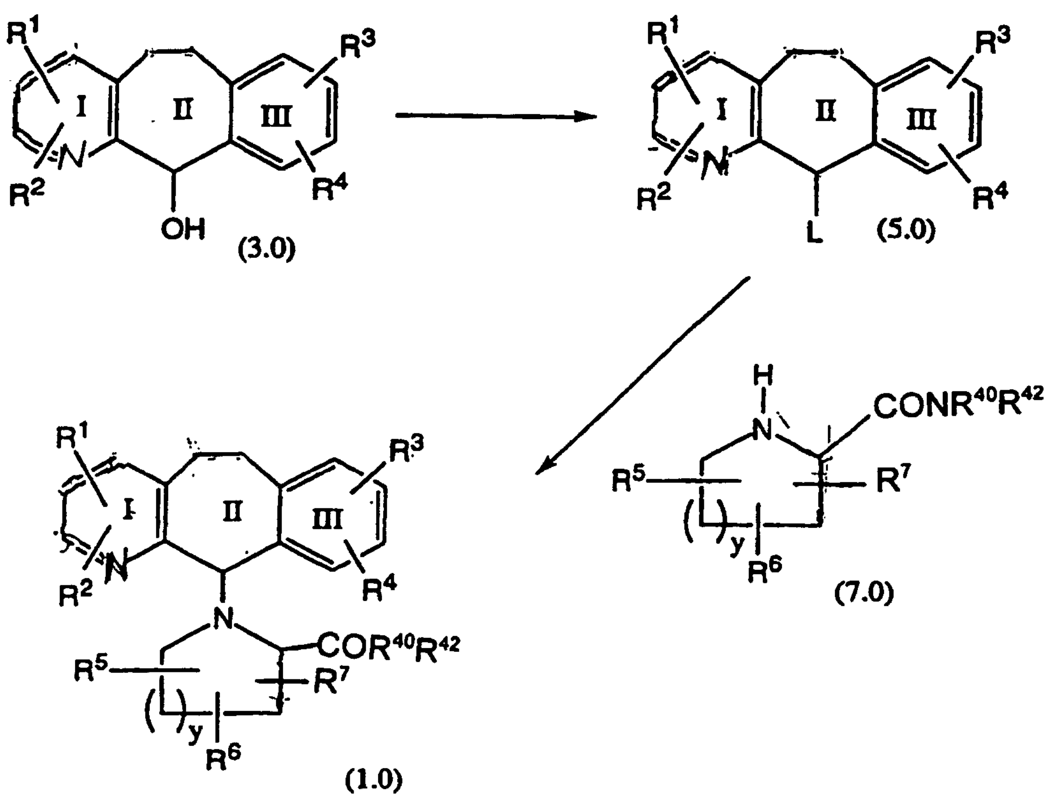

(3.0) (5.0) (7.0) (1.0)

wherein L represents a leaving group such as halo, preferably chloro or a leaving group such as o-tosyl and o-mesyl; $R^1$, $R^2$, $R^3$, $R^4$, $R^5$, $R^6$, $R^7$ $R^{40}$, $R^{42}$ and y are as defined hereinbefore.

Referring to the Scheme I, compounds of formula (5.0) can be prepared by reacting the compounds of formula (3.0) with a halogenating agent or a sulfonylating agent in the presence of a suitable base, and optional aprotic solvent, in amounts and under conditions effective to give compounds (5.0). Suitable bases include organic bases such as pyridine and triethylamine; or inorganic bases of alkali and alkaline earth metals including carbonates such as sodium, lithium, potassium and cesium carbonates, hydroxides such as sodium and potassium hydroxides; hydrides such as sodium or potassium hydride; and sodium t-butoxide, preferably sodium hydride. Suitable aprotic solvents include ethers, DMF, DMSO, THF, DME and mixtures thereof, preferably DMF. Preferably the halogenating agent is a chlorinating agent, such as thionyl chloride. The sulfonylating can be sulfonyl chloride, methane sulfonyl chloride or toluene sulfonyl chloride. The amounts of the halogenating agent or the sulfonylating agent can range from about one to about 10 moles per mole of compound (3.0). Temperatures can range from 0° to 50°C, or reflux of the reaction mixture.

The desired tricylic piperidinyl compounds of formula (1.0) can be prepared by reacting the compounds of formula (5.0) with a suitably substituted pyrrolidine or azetidine compound of formula (7.0) in the presence of a suitable base and optional aprotic solvent, such as those described above, to give compounds (1.0). The amounts of the substituted pyrrolidine or azetidine compound of formula (7.0) to compound (5.0) can range from about one to about 10 moles per mole of compound (5.0) Temperatures can range from about room temperature to about 80°C.

The tricylic compounds of fomula (1.0) can be isolated from the reaction mixture using conventional procedures, such as, for example, extraction of the reaction mixture from water with organic solvents, evaporation of the organic solvents, followed by chromatography on silica gel or other suitable chromatographic media.

The compound of formula (1.0) wherein T = -CO- and Z = $-NR^{40}R^{42}$ wherein $R^{40}$ and $R^{42}$ are defined hereinbefore (i.e. an amide) can be prepared in accordance with Scheme 2.

Scheme 2

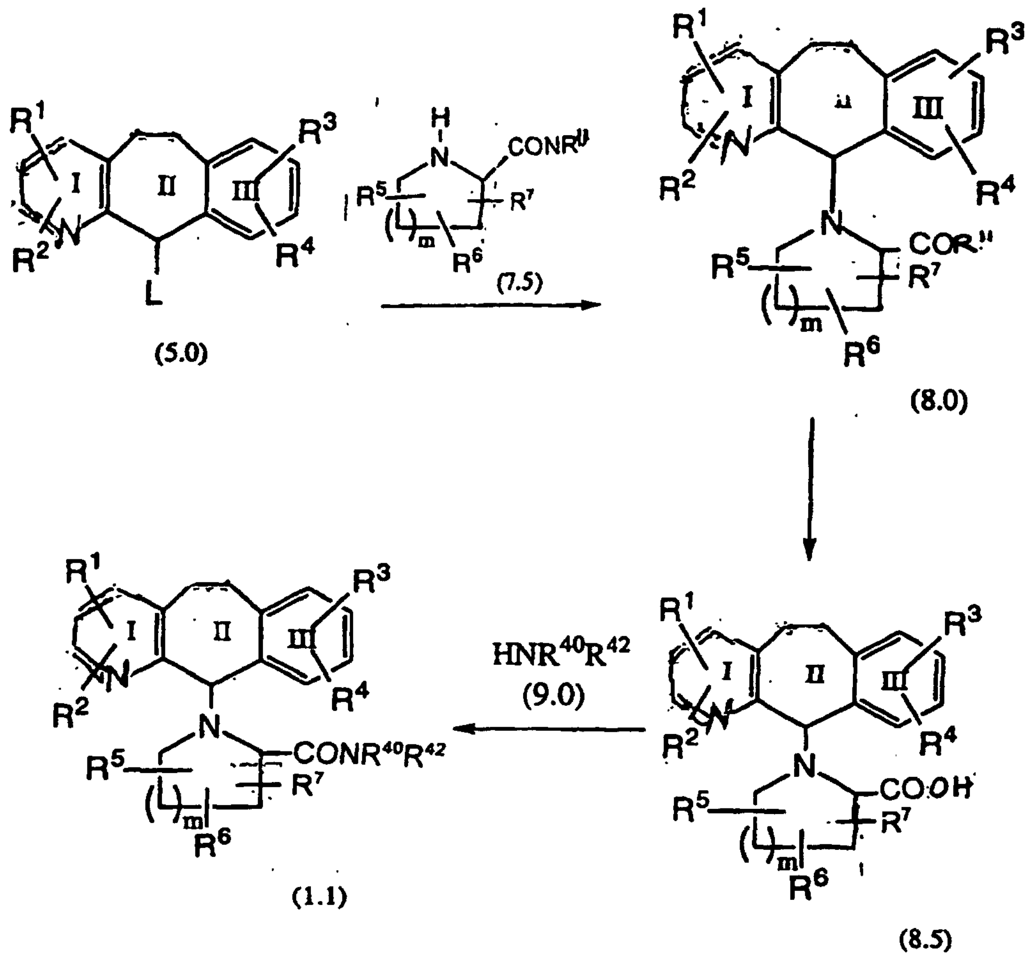


(5.0) (7.5) (8.0) (8.5) (1.1)

wherein L represents a leaving group, preferably chloro; $R^1$, $R^2$, $R^3$, $R^4$, $R^5$, $R^6$, $R^7$, $R^{11}$ , $R^{40}$, $R^{42}$, m and n are as defined hereinbefore.

Referring to the Scheme 2, compounds of formula (8.0) can be prepared by reacting the compounds of formula (5.0) with a piperdinyl carboxylic acid ester of formula (7.5 ) in the presence of a base and optional aprotic solvent, in amounts and under conditions effective to give compounds (8.0). Suitable bases and aprotic solvents are described hereinbefore. The amounts of compound (7.5) can range from about 1 to about 10 moles per mole of compound (5.0). Temperatures can range from room temperature to about 80°C. Compound (8.0) can be isolated as described hereinbefore.

Carboxylic acid compounds of formula (8.5) can be prepared by hydrolyzing carboxylic acid ester (8.0) with an excess amount of acid or base. Suitable acids include inorganic acids, organic acids or a mixture thereof. Inorganic acids include hydrogen chloride, hydrogen bromide, sulfuric acid, nitric acid, phosphoric acid, perchloric acid and the like. Organic acids include acetic, citric, formic, maleic, tartaric, methanesulfonic acid and arylsulfonic acids. Suitable bases, such as sodium hydroxide, or lithium hydroxide in an aqueous alcohol, have been described hereinbefore. The temperature can range from about 0°C to about 100°C.

The desired amide compounds of formula (1.1) can be prepared by reacting the compounds of formula (8.5) with a suitable amine of formula (9.0) in the presence of a base and a suitable aprotic solvent effective to give amide compound (1.1). Suitable bases and aprotic solvents are described hereinbefore. The amounts of amine (9.0) can

range from about 1 to about 10 moles per mole of carboxylic acid (8.5). Temperatures can range from 0° to 100°C. Compound (1.1) can be isolated as described hereinbefore.

Compounds of the present invention and preparative starting materials therof, are exemplified by the following examples.

Example 1.

Step A. 1-(3-Bromo-8-chloro-6,11-dihydro-5H-benzo[5,6]cyclohepta[1,2-b]pyridine-11-yl)-N-2-pyrrolidine methyl ester

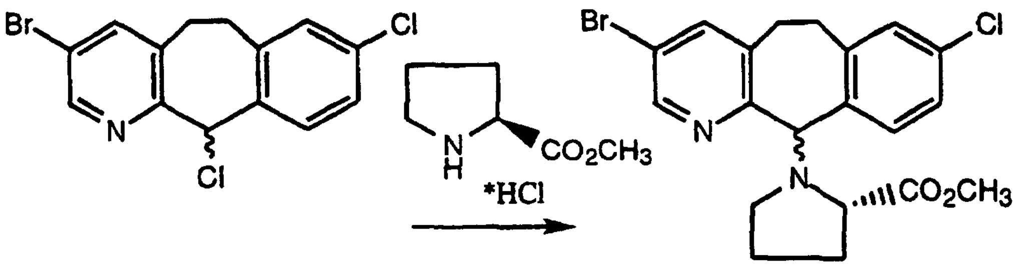


A mixture of 3-bromo-8,11-dichloro-6,11-dihydro-5H-benzo[5,6]cyclohepta[1,2-b]pyridine (1.05 g, 3.06 mmole) , proline methyl ester hydrochloride (1.52 g 9.18 mmole) and N-methyl morpholine (1.85 g, 18.32 mmole) in DMF (15 mL) is heated at 85°C overnight. The reaction mixture is evaporated to dryness, extracted with $CH_2Cl_2$ (100 mL), washed with water (2 x100 mL), the organic extract is dried over $MgSO_4$ and the solvent evaporated to give an oily residue. The oily residue is flash chromatographed on a silica gel column eluting with hexane-15% ethyl acetate to give 0,78 g of the title compound a foam. Partial PMR ($CDCl_3$, 200 MHz), 8.3 (s, 1H), 7.5 (d, 1H), 7-7.2 (m, 3H), 4.5 (s, 1H), 3.2 (s, 3H).

Step B. 1-(3-Brorno-8-chloro-6,11-dihydro-5H-benzo[5,6]cyclohepta[1,2-b]pyridine-11-yl)-N-(3-pyridinylmethyl) 2-pyrrolidine carboxamide

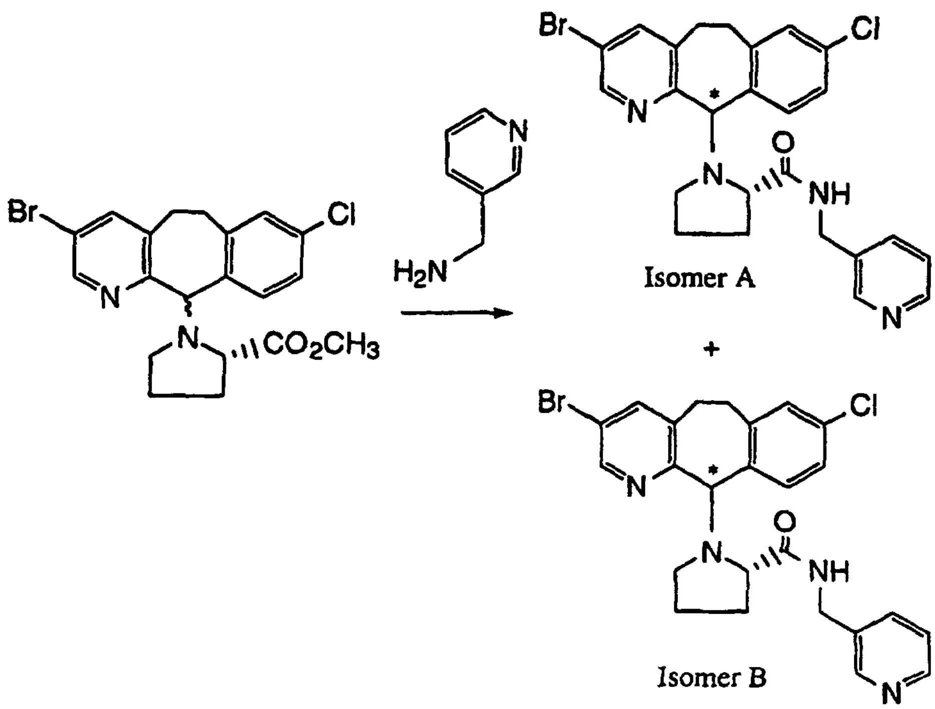
Isomer A

Isomer B

The title compound of Example 1, Step A (0.43 g, 9.1 mmole) and 3-aminomethylpyridine (0.196 g, 18.12 mmole) are heated at 130°C overnight. The residue is chromatographed on a silica gel column eluting with $CH_2Cl_2$-3% ($CH_3OH$-10% conc $NH_4OH$). and separated to give the title compounds:

Isomer A, 0.062 g , Mass Spec. $MH^+$ 513 (FAB); partial PMR (CDCl3, 200MHz), 8.45 (d, 1H), 8.4 (s, 1H), 8.3 (s, 1H), 7-7.4 (m, 6H), 4.68 (s, 1H) FPT $IC_{50}$ = 0.059 μM

Isomer B, 0.042 g, Mass spec. $MH^+$513, partial PMR (CDCl3, 200 MHz), 8.55 (d, 1H), 8.4 (s, 1H), 8.35 (s, 1H), 6.8-7.6 (m, 6H). FPT $IC_{50}$ = 0.14 μM

Example 2.

Step A. 1-(3,10-Dibrorno-8-chloro-6,11-dihydro-5H-benzo[5,6]cyclohepta[1,2-b]pyridine-11-yl)-N-2-pyrrolidine methyl ester

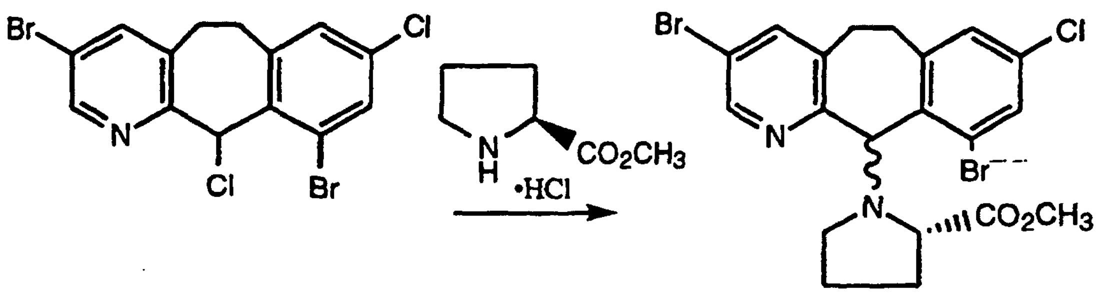


A mixture of 3,10-dibromo-8,11-dichloro-6,11-dihydro-5H-benzo[5,6]cyclohepta[1,2-b]pyridine (0.5 g, 1.18 mmole) , proline methyl ester hydrochloride (0.59 g 3.55 mmole) and N-methyl morpholine (0.72 g, 7.11 mmole) in DMF (10 ML) is heated at 85°C for one hour. The reaction mixture is evaporated to dryness, extracted with $CH_2Cl_2$ (100 mL) and washed with water (2 x100 mL). The organic extract is dried over $MgSO_4$ and the solvent evaporated, leaving an oily residue which is flash chromatographed on a silica gel column eluting with hexane-15% ethyl acetate to give 0.43 g of the title compound as a foam. Partial PMR ($CDCl_3$, 200 MHz), 8.4 (d, 1H), 7.45 (d, 1H), 7.4 (d, 1H), 7.12 (d, 1H),5.56 (s, 1H), 5.01 (m, 1H), 3.55 (m, 1H), 3.23 (s, 3H).

Step B. 1-(3,10-dibromo-8-chloro-6,11-dihydro-5H-benzo[5,6]cyclohepta[1,2-b]pyridine-11-yl)-N-(3-pyridinylmethyl)-2-pyrrolidine carboxamide

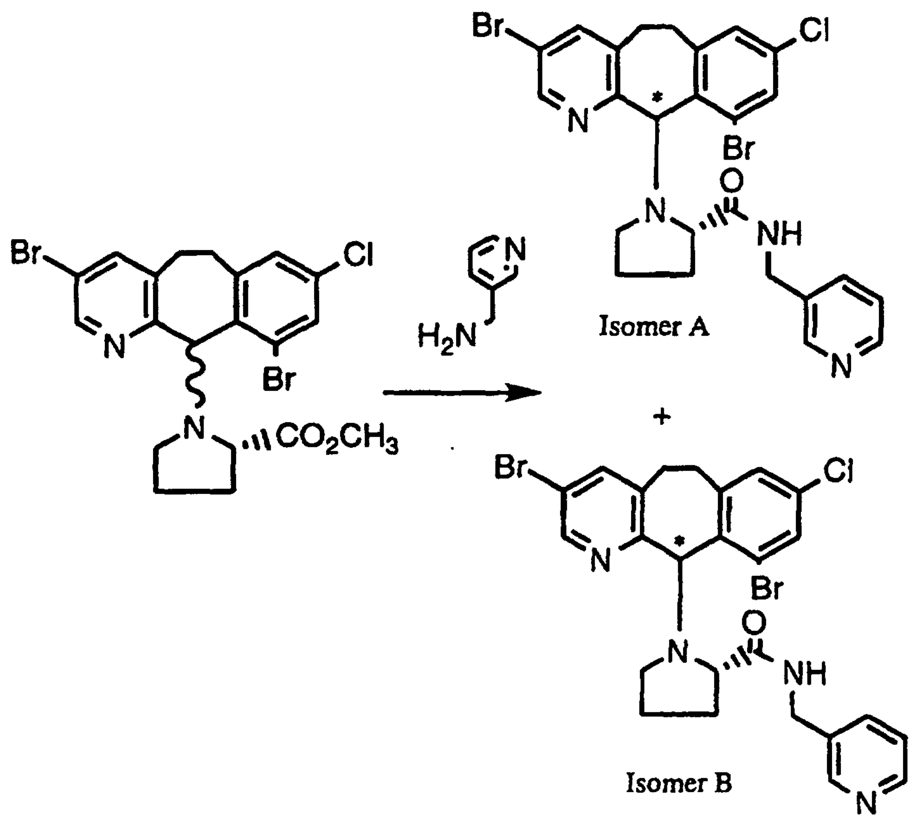

Isomer A

Isomer B

The compound of Example 2, Step A (0.36 g ) is dissolved in ethanol (10 mL) and heated at 80 °C with 1N LiOH (aqueous, 4 mL) overnight.. The pH is adjusted to 4 with 1N HCl and the solution evaporated to dryness. The product is dissolved in DMF (10 mL) and NMM (0.32 mL), and HOBT (0.187 g), DEC (0.265 g), and 3-amino methyl pyridine (0.16 mL) are added. The reaction mixture is stirred over the weekend, evaporated to dryness, the residue extracted in $CH_2Cl_2$ (100 mL) and with brine (2 x 100 mL). The organic extract is dried over $MgSO_4$, evaporated to dryness, and chromatographed on a Chiralpak® AD HPLC analytical chiral column (amylose tris(3,5-dimethylphenyl carbamate) coated on a 10 μM silica-gel substrate, trademark of of Chiral Technologies, Exton, Pennsylvania)), using as the eluting solvent,eluting with 80% hexane/isopropanol (containing 0.25 % diethylamine) to give the title compounds:

Isomer A (0.124 g) as a foam, Mass Spec. $MH^+$ 591 (FAB); partial PMR (CDCl3, 400MHz), 8.58 (d, 1H), 8.45 (s, 2H), 8.3 (s, 1H), 7.55 (s, 1H), 7.45(m, 2H),7.28 (m, 2H), 6.82 (s, 1H), 6.81 (t, 1H), 5.72 (s, 1H) FPT Inhibition: 15% @ 0.3 μM

Isomer B, 0.165g, Mass spec. $MH^+$591, partial PMR (CDCl3, 400 MHz), 8.59 (d, 1H), 8.4 (m, 2H), 7.48 (s, 1H), 7.35 (m, 1H), 7.1-7.3 (m, 3H), 6.9 (t, 1H), 5.6 (s, 1H).

FPT $IC_{50}$ = 0.0052 μM

Example 3.

Step A. 1-(3-bromo-8-chloro-6,11-dihydro-5H-benzo[5,6]cyclohepta[1,2-b]pyridine-11-yl)-N-2-azetidine methyl ester

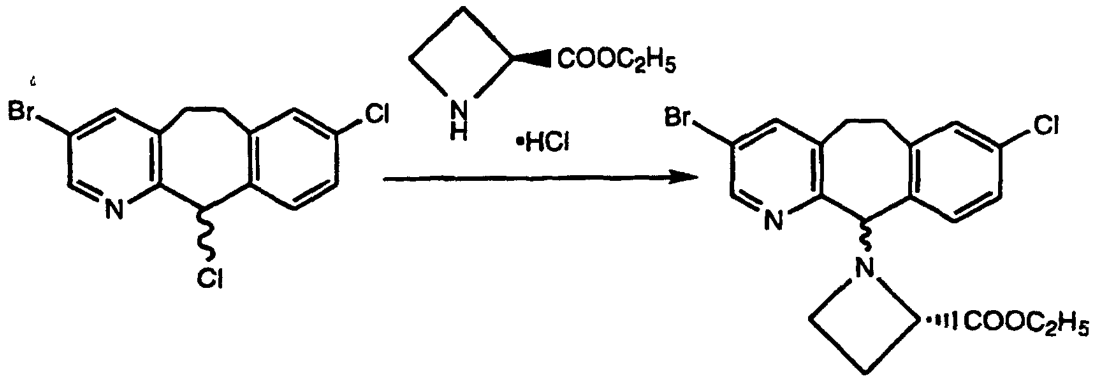


A mixture of 3-bromo-8,11-dichloro-6,11-dihydro-5H-benzo[5,6]cyclohepta[1,2-b]pyridine (1.05 g, 3.06 mmole), azetidine ethyl ester hydrochloride (1.52 g 6.02 mmole) and N-methyl morpholine (1.85 g, 18.32 mmole) in DMF (15 ML) is heated at 85°C overnight. The reaction mixture is evaporated to dryness, extracted with $CH_2Cl_2$ (100 mL) and washed with water (2 x100 mL). The organic extract is dried over $MgSO_4$ and the solvent is evaporated to give oily residue which is flash chromatographed on a silica gel column eluting with hexane-15% ethyl acetate, to give 0.72 g of the title compound as a foam. Partial PMR ($CDCl_3$, 200 MHz), 8.3 (s, 1H), 7.5 (d, 1H), 7-7.2 (m, 3H), 4.5 (s, 1H), 3.2 (s, 3H).

Step B. 1-(3-bromo-8-chloro-6,11-dihydro-5H-benzo[5,6]cyclohepta[1,2-b]pyridine-11-yl)-N-(3-pyridinylmethyl) 2-azetidine carboxamide

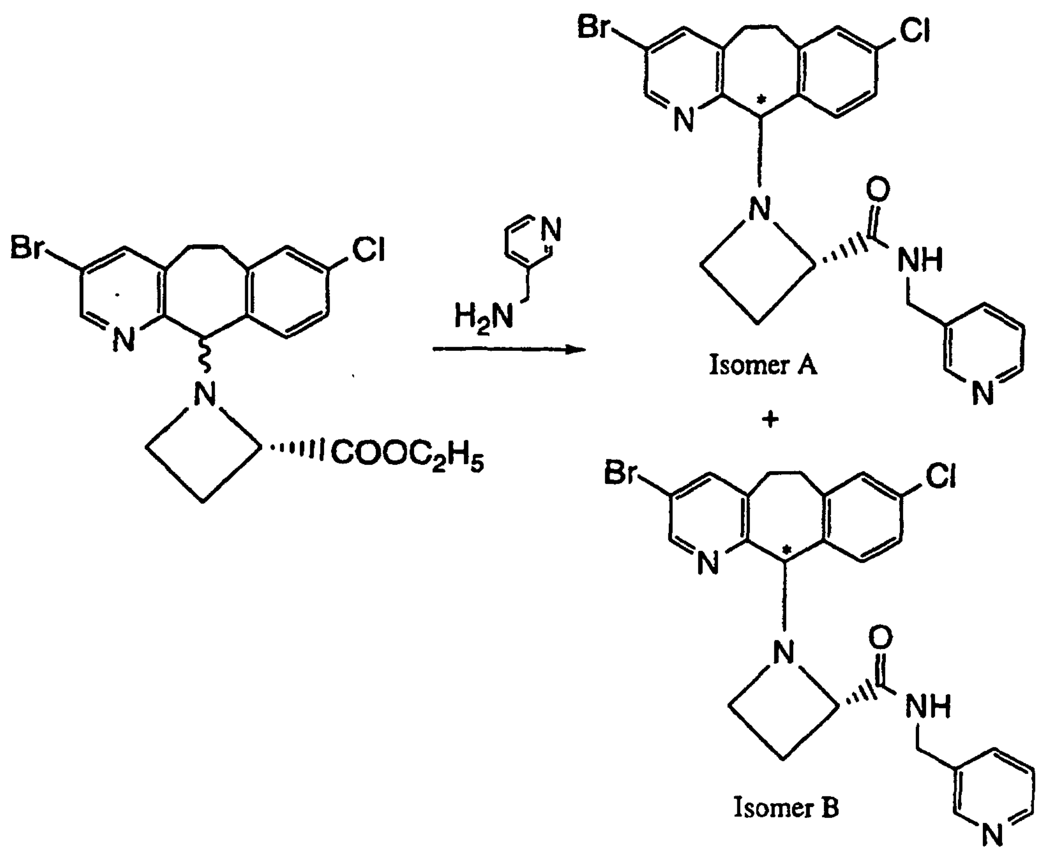


The compound of Example 3, Step A (0.7 g ) is dissolved in ethanol (10 mL) and heated at 80°C with 1N LiOH (aqueous, 3 mL) ovemight. The pH is adjusted to 4 with 1N HCl and the solution evaporated to dryness. The product is dissolved in DMF (10 mL) and NMM (0.32 mL), and HOBT (0.187 g), DEC (0.265 g) and 3-aminomethyl pyridine (0.16 mL) are added. The reaction mixture is stirred over the week end at room temperature, evaporated to dryness, and the residue extracted in $CH_2Cl_2$ (100 mL). The organic extract is washed with brine (2 x 100 mL), dried over $MgSO_4$, evaporated to dryness and the product is chromatographed on a Chiralpak® AD HPLC analytical chiral column eluting with 80% hexane/Isopropanol (containing 0.25 % diethylamine) to give the title compounds:

Isomer A (0.113 g) as a foam, Mass Spec. $MH^+$ 499 (FAB); partial PMR (CDCl3, 400MHz), 8.58 (d, 1H), 8.45 (s, 2H), 8.3 (s, 1H), 7.55 (s, 1H), 7.45(m, 2H),7.28 (m, 2H), 6.82 (s, 1H), 6.81 (t, 1H), 5.72 (s, 1H) FPT $IC_{50}$ = 1.05 μM

Isomer B (0.148g) as a foam, Mass spec. $MH^+$499, partial PMR ($CDCl_3$, 400 MHz), 8.59 (d, 1H), 8.4 (m, 2H), 7.48 (s, 1H), 7.35 (m, 1H), 7.1-7.3 (m, 3H), 6.9 (t, 1H), 5.6 (s, 1H)

FPT Inhibition: 17% @0.1 μM

PREPARATION OF STARTING MATERIALS

Starting materials useful in preparing the compounds of the present invention are exemplified by the following preparative examples, which should not be construed to limit the scope of the disclosure. The tricylic compounds (3.0) and substituted piperidinyl compounds (7.0) used as starting materials are known in the art and/or can be prepared using known methods, such as taught in U.S. Patents 5,089,496; 5,151,423; 4,454,143; 4,355,036; PCT /US94/11390 (WO95/10514); PCT/US94/11391 (WO 95/10515); PCT/US94/11392 (WO95/10516); Stanley R. Sandler and Wolf Karo, Organic Functional Group Preparations, 2nd Edition, Academic Press, Inc., San Diego, California, Vol. 1-3,

(1983); in J. March, Advanced Organic Chemistry, Reactions & Mechanisms, and Structure, 3rd Edition, John Wiley & Sons, New York, 1346 pp. (1985); A. J. Boulton and A. McKillop (Eds.), Comprehensive Heterocyclic Chemistry, Volume 7, Four Membered Rings With One Nitrogen Atom, Pergamon Press, Elmsford, New York, (1960-1985); A. J. Boulton and A. McKillop (Eds.), Comprehensive Heterocyclic Chemistry, Volume 4, Part 3, Five Membered Rings With One Nitrogen Atom, Pergamon Press, Elmsford, New York, (1960-1985); J. Am. Chem. Soc. 80, pg. 970 (1958); JOC 33, 3637 (1968); Tetra. Letters, pp. 381-382 (1995); Helvetics. Chem. Acta, 59 (6), pp. 1917-24 (1976); and J. Med. Chem., 33, 71-77 (1990). The starting materials may also be prepared as taught in copending U.S. Application Serial No. 08/410,187 filed March 24, 1995, copending U.S. Application Serial No. 08/577,951 filed December 22, 1995, and copending U.S. Application Serial No. 08/615,760 filed March 13, 1996; the disclosures being incorporated herein by reference. Alternative mechanistic pathways and analogous structures within the scope of the invention may be apparent to those skilled in the art.

For example, the pyrrolidine compounds of formula (7.0), wherein T = -CO- can be prepared by initially preparing a pyrole compound substituted with the requisite 2- or 3-$(CH_2)_nCOZ$ moiety, together any optional -$R^5$, -$R^6$, -$R^7$ and/or -$R^8$ moieties as described in the references cited above and other known, art. The 2- or 3-substituted pyrole compound can subsequently be reduced using conventional reduction procedures.

Pharmaceutical Dosage Form Examples

| EXAMPLE A-Tablets | | | |
|---|---|---|---|
| No. | Ingredients | mg/tablet | mg/tablet |
| 1. | Active compound | 100 | 500 |
| 2. | Lactose USP | 122 | 113 |
| 3. | Corn Starch, Food Grade, as a 10% paste in Purified Water | 30 | 40 |
| 4. | Com Starch, Food Grade | 45 | 40 |
| 5. | Magnesium Stearate | 3 | 7 |
| | Total | 300 | 700 |

Method of Manufacture

Mix Item Nos. 1 and 2 in a suitable mixer for 10-15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

| EXAMPLE B-Capsules | | | |
|---|---|---|---|
| No. | Ingredient | mg/capsule | mg/capsule |
| 1. | Active compound | 100 | 500 |
| 2. | Lactose USP | 106 | 123 |
| 3. | Com Starch, Food Grade | 40 | 70 |
| 4. | Magnesium Stearate NF | 7 | 7 |
| | Total | 253 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

## Claims

1. A compound of the formula:

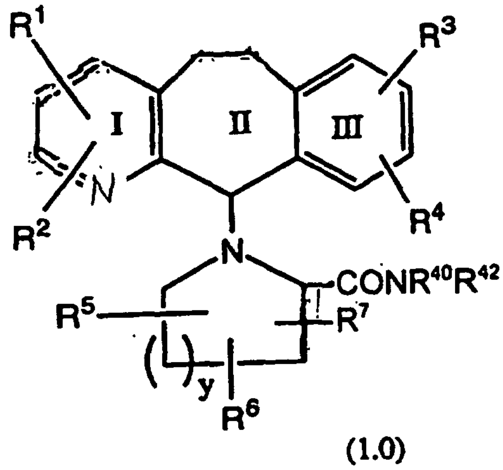

(1.0)

or a pharmaceutically acceptable salt or solvate thereof, wherein:

$R^1$ and $R^4$ are H and $R^2$ and $R^3$ are halo selected from chloro and bromo; or $R^1$ is H and $R^2$, $R^3$ and $R^4$ are halo selected from chloro and bromo.

$R^5$ and $R^6$ (y = 0) or $R^5$, $R^6$ and $R^7$ (y = 1) each independently represents H, $-CF_3$, $-COR^{10}$, alkyl or aryl, said alkyl or aryl optionally being substituted with $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$, $OPO_3R^{10}$ or one of $R^5$, $R^6$ and $R^7$ can be taken in combination with $R^{40}$ as defined below to represent $-(CH_2)_r-$ wherein r is 1 to 4 which can be substituted with $C_{1-6}$ alkyl, $C_{1-6}$ alkoxy, $-CF_3$ or aryl, or $R^5$ is combined with $R^6$ or $R^7$ to represent = O or = S;

$R^{10}$ independently represents H, alkyl, cycloalkyl, cycloalkylalkyl, heteroaryl, aryl, aralkyl or $-NR^{40}R^{42}$;

$R^{11}$ represents alkyl or aryl;

$R^{40}$ and $R^{42}$ independently represent H, aryl, alkyl, aralkyl, heteroaryl, heteroarylalkyl, heterocycloalkyl, heterocycloalkylalkyl, heteroalkyl, cycloalkyl, cycloalkylalkyl, alkenyl and alkynyl;

y is 0 (zero) or 1;

and wherein:

alkyl-(including the alkyl portions of alkoxy, alkylamino and dialkylamino)-represents straight and branched carbon chains and contains from one to twenty carbon atoms, wherein said alkyl group may be optionally and independently substituted with one, two, three or more optional substituents;

alkenyl-represents straight and branched carbon chains having at least one carbon to carbon double bond and containing from 2 to 12 carbon atoms, wherein said alkenyl group may be optionally and independently substituted with one, two, three or more optional substituents;

alkynyl-represents straight and branched carbon chains having at least one carbon to carbon triple bond and containing from 2 to 12 carbon atoms, wherein said alkynyl group may be optionally and independently substituted with one, two, three or more optional substituents;

aryl (including the aryl portion of aralkyl)-represents a carbocyclic group containing from 6 to 15 carbon atoms and having at least one aromatic ring, wherein said aryl group optionally can be fused with aryl, cycloalkyl, heteroaryl or heterocycloalkyl rings; and wherein any of the available substitutable carbon and nitrogen atoms in said aryl group and/or said fused ring(s) may be optionally and independently substituted with one, two, three or more optional substituents;

aralkyl - represents an alkyl group, as defined above, wherein one or more hydrogen atoms of the alkyl moiety have been substituted with one or more aryl groups; wherein said aralkyl group may be optionally and independently substituted with one, two, three or more optional substituents;

cycloalkyl - represents saturated carbocyclic rings branched or unbranched of from 3 to 20 carbon atoms, wherein said cycloalkyl group may be optionally and independently substituted with one, two, three or more optional substituents;

cycloalkylalkyl - represents an alkyl group, as defined above, wherein one or more hydrogen atoms of the alkyl moiety have been substituted with one or more cycloalkyl groups; wherein said cycloalkylalkyl group may be optionally and independently substituted with one, two, three or more optional substituents;
heteroaryl-represents cyclic groups having at least one heteroatom selected from O, S and N, said heteroatoms(s) interrupting a carbocyclic ring structure and having a sufficient number of delocalized pi electrons to provide aromatic character, with the aromatic heterocyclic groups containing from 2 to 14 carbon atoms, wherein said heteroaryl group optionally can be fused with one or more aryl, cycloalkyl, heteroaryl or heterocycloalkyl rings; and wherein any of the available substitutable carbon or nitrogen atoms in said heteroaryl group and/or said fused ring(s) may be optionally and independently substituted with one, two, three or more optional substituents;
heteroarylalkyl - represents an alkyl group, as defined above, wherein one or more hydrogen atoms have been replaced by one or more heteroaryl groups; wherein said heteroarylalkyl group may be optionally and independently substituted with one, two, three or more optional substituents;
heterocycloalkyl - represents a saturated, branched or unbranched carbocyclic ring containing from 3 to 15 carbon atoms, which carbocyclic ring is interrupted by 1 to 3 heteroatoms selected from -O-, -S- and -N-, wherein optionally, said ring may contain one or two unsaturated bonds which do not impart aromatic character to the ring; and wherein any of the available substitutable carbon and nitrogen atoms in the ring may be optionally and independently substituted with one, two, three or more optional substituents; and "optional substituents" are selected from halo, alkyl, aryl, alkoxy, amino, alkylamino, cyano, $-CF_3$, dialkylamino, hydroxy, oxy, phenoxy, $-OCF_3$, heterocycloalkyl, $-SO_2NH_2$, $-NHSO_2R^{10}$, $-SO_2NHR^{10}$, $SO_2R^{10}$, $-SOR^{10}$, $-SR^{10}$, $-NHSO_2$, $-NO_2$, $-CONR^{10}$, $-NCOR^{10}$ or $-COOR^{10}$.

**2.** The compound of Claim 1 wherein $R^2$ is halo in the 3-position and $R^3$ is halo in the 8-position.

**3.** The compound of Claim 1 wherein $R^2$ is Br in the 3-position and $R^3$ is Cl in the 8-position.

**4.** The compound of Claim 1 wherein $R^1$ is H and $R^2$ $R^3$ and $R^4$ are halo selected from chloro and bromo.

**5.** The compound of Claim 1 wherein $R^2$ is halo in the 3-position, $R^3$ is halo in the 8-position and $R^4$ is halo in the 10-position.

**6.** The compound of Claim 1 wherein $R^2$ is bromo in the 3-position, $R^3$ is chloro in the 8-position and $R^4$ is bromo in the 10-position.

**7.** The compound of any preceding claim wherein $R^{40}$ is H; and $R^{42}$ is 3-pyridylmethyl.

**8.** The compound of Claim 1 selected from
1-(3-bromo-8-chloro-6,11-dihydro-5H-benzo[5,6]cyclohepta[1,2-b]pyridine-11-yl)-N-(3-pyridinylmethyl) 2-pyrrolidine carboxamide;
1-(3,10-dibromo-8-chloro-6,11-dihydro-5H-benzo[5,6] cyclohepta [1,2-b]pyridine-11-yl)-N-(3-pyridinylmethyl)-2-pyrrolidine carboxamide; and
1-(3-bromo-8-chloro-6,11-dihydro-5H-benzo[5,6]cyclohepta[1,2-b]pyridine-11-yl)-N-(3-pyridinylmethyl) 2-azetidine carboxamide

**9.** The compound of Claim 8 which is selected from

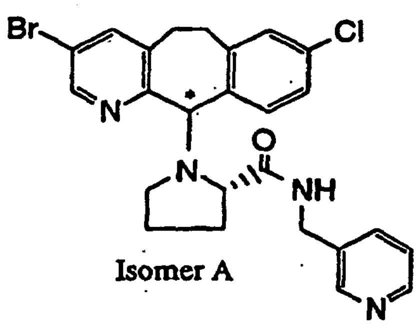

Isomer A

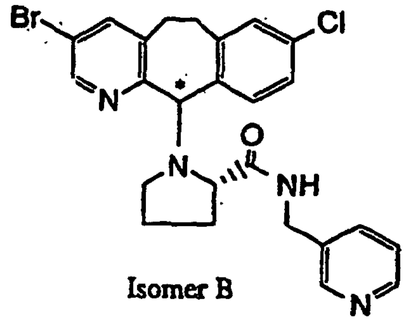

Isomer B

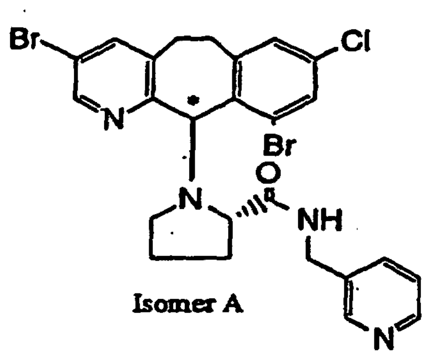

Isomer A

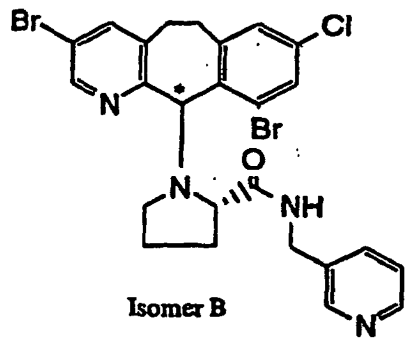

Isomer B

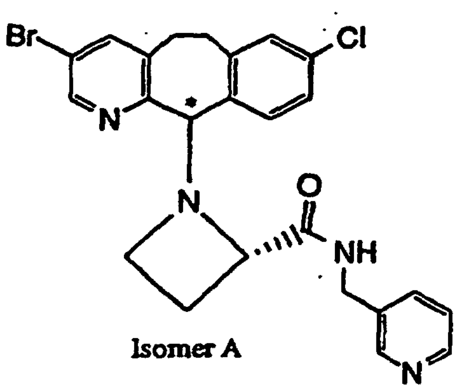

Isomer A

and

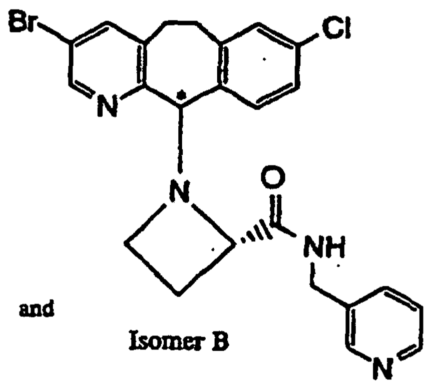

Isomer B

or a pharmaceutically acceptable salt thereof.

**10.** The compound of Claim 9 which is

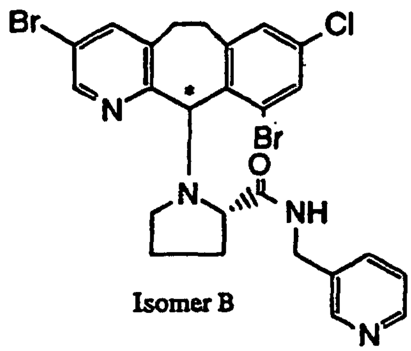

Isomer B

11. A pharmaceutical composition for inhibiting the abnormal growth of cells comprising an effective amount of compound of any of Claims 1 to 10 in combination with a pharmaceutically acceptable carrier.

12. A compound according to any of Claims 1 to 10 for use in inhibiting the abnormal growth of cells.

13. A compound according to Claim 12 wherein the cells inhibited are tumor cells expressing an activated ras oncogene.

14. A compound according to Claim 12 wherein the cells inhibited are pancreatic tumor cells, lung cancer cells, myeloid leukemia tumor cells, thyroid follicular tumor cells, myelodysplastic tumor cells, epidermal carcinoma tumor cells, bladder carcinoma tumor cells or colon tumors cells.

15. A compound according to any of Claims 12 to 14 wherein the inhibition of the abnormal growth of cells occurs by the inhibition of ras farnesyl protein transferase.

16. A compound according to any of Claims 12 to 15 wherein the inhibition is of tumor cells wherein the Ras protein is activated as a result of oncogenic mutation in genes other than the Ras gene.

## Patentansprüche

1. Verbindung mit der Formel:

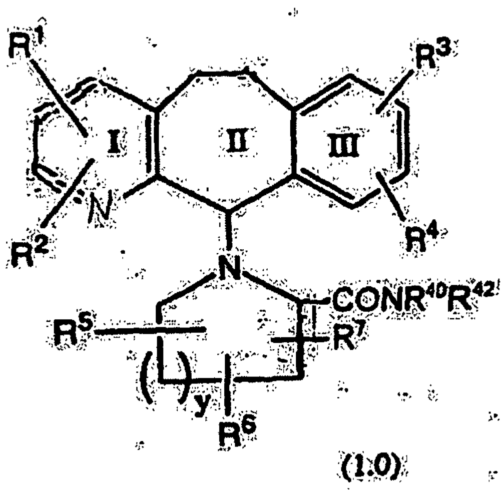

(1.0)

oder ein pharmazeutisch annehmbares Salz oder Solvat davon, worin
$R^1$ und $R^4$ H sind und $R^2$ und $R^3$ Halogen ausgewählt aus Chlor und Brom sind; oder $R^1$ H ist und $R^2$, $R^3$ und $R^4$

Halogen ausgewählt aus Chlor und Brom sind;
$R^5$ und $R^6$ (y = 0) oder $R^5$, $R^6$ und $R^7$ (y = 1) jeweils unabhängig für H, $-CF_3$, $-COR^{10}$, Alkyl oder Aryl stehen, wobei das Alkyl oder Aryl gegebenenfalls mit $-OR^{10}$, $-SR^{10}$ $-S(O)_tR^{11}$, $-NR^{10}COOR^{11}$, $-N(R^{10})_2$, $-NO_2$, $-COR^{10}$ $-OCOR^{10}$, $-OCO_2R^{11}$, $-CO_2R^{10}$, $OPO_3R^{10}$ substituiert ist, oder einer von $R^5$, $R^6$ und $R^7$ in Kombination mit $R^{40}$ wie nachfolgend definiert $-(CH_2)r-$ wiedergeben kann, wobei r 1 bis 4 ist, und mit $C_1$-$C_6$-Alkyl, $C_1$-$C_6$-Alkoxy, $-CF_3$ oder Aryl substituiert sein kann, oder $R^5$ mit $R^6$ oder $R^7$ kombiniert ist, um =O oder =S wiederzugeben;
$R^{10}$ unabhängig für H, Alkyl, Cycloalkyl, Cycloalkylalkyl, Heteroaryl, Aryl, Aralkyl oder $-NR^{40}R^{42}$ steht;
$R^{11}$ für Alkyl oder Aryl steht;
$R^{40}$ und $R^{42}$ unabhängig für H, Aryl, Alkyl, Aralkyl, Heteroaryl, Heteroarylalkyl, Heterocycloalkyl, Heterocycloalkylalkyl, Heteroalkyl, Cycloalkyl, Cycloalkylalkyl, Alkenyl und Alkinyl stehen;
y 0 (Null) oder 1 ist;
und worin:

Alkyl (einschließlich der Alkylanteile von Alkoxy, Alkylamino und Dialkylamino) für geradkettige und verzweigte Kohlenstoffketten steht und ein bis zwanzig Kohlenstoffatome enthält, wobei die Alkylgruppe gegebenenfalls und unabhängig mit einem, zwei, drei oder mehr optionalen Substituenten substituiert sein kann;

Alkenyl für geradkettige und verzweigte Kohlenstoffketten mit mindestens einer Kohlenstoff-Kohlenstoff-Doppelbindung steht und 2 bis 12 Kohlenstoffatome enthält, wobei die Alkenylgruppe gegebenenfalls und unabhängig mit einem, zwei, drei oder mehr optionalen Substituenten substituiert sein kann;

Alkinyl für geradkettige und verzweigte Kohlenstoffketten mit mindestens einer Kohlenstoff-Kohlenstoff-Dreibindung steht und 2 bis 12 Kohlenstoffatome enthält, wobei die Alkinylgruppe gegebenenfalls und unabhängig mit einem, zwei, drei oder mehr optionalen Substituenten substituiert sein kann;

Aryl (einschließlich des Arylanteils von Aralkyl) für eine carbocyclische Gruppe steht, die 6 bis 15 Kohlenstoffatome enthält und mindestens einen aromatischen Ring aufweist, wobei die Arylgruppe gegebenenfalls mit Aryl-, Cycloalkyl-, Heteroaryl- oder Heterocycloalkylringen kondensiert sein kann, und wobei irgendwelche der verfügbaren substituierbaren Kohlenstoff- und Stickstoffatome in der Arylgruppe und/oder dem kondensierten Ring bzw. den kondensierten Ringen gegebenenfalls und unabhängig mit einem, zwei, drei oder mehr optionalen Substituenten substituiert sein kann;

Aralkyl für eine Alkylgruppe wie oben definiert steht, bei der ein oder mehrere Wasserstoffatome der Alkyleinheit durch eine oder mehrere Arylgruppen substituiert worden sind, wobei die Aralkylgruppe gegebenenfalls und unabhängig mit einem, zwei, drei oder mehr optionalen Substituenten substituiert sein kann;

Cycloalkyl für gesättigte carbocyclische, verzweigte oder unverzweigte Ringe mit 3 bis 20 Kohlenstoffatomen steht, bei der die Cycloalkylgruppe gegebenenfalls und unabhängig mit einem, zwei, drei oder mehr optionalen Substituenten substituiert sein kann;

Cycloalkylalkyl für eine Alkylgruppe wie oben definiert steht, bei der ein oder mehrere Wasserstoffatome der Alkyleinheit durch eine oder mehrere Cycloalkylgruppen substituiert worden sind, wobei die Cycloalkylalkylgruppe gegebenenfalls und unabhängig mit einem, zwei, drei oder mehr optionalen Substituenten substituiert sein kann;

Heteroaryl für cyclische Gruppen mit mindestens einem Heteroatom ausgewählt aus 0, S und N steht, wobei das Heteroatom/die Heteroatome eine carbocyclische Ringstruktur unterbricht bzw. unterbrechen, und die eine ausreichende Anzahl delokalisierter Π-Elektronen aufweisen, um aromatischen Charakter zu liefern, wobei die aromatischen heterocyclischen Gruppen 2 bis 14 Kohlenstoffatome enthalten, wobei die Heteroarylgruppe gegebenenfalls an einen oder mehrere Aryl-, Cycloalkyl-, Heteroaryl- oder Heterocycloalkylringe kondensiert sein kann; und wobei irgendwelche der verfügbaren Kohlenstoff- oder Stickstoffatome in der Heteroarylgruppe und/oder dem kondensierten Ring bzw. den kondensierten Ringen gegebenenfalls und unabhängig mit einem, zwei, drei oder mehr optionalen Substituenten substituiert sein kann;

Heteroarylalkyl für eine Alkylgruppe wie oben definiert steht, bei der ein oder mehrere Wasserstoffatome durch eine oder mehrere Heteroarylgruppen substituiert worden sind, wobei die Heteroarylgruppe gegebenenfalls und unabhängig mit einem, zwei, drei oder mehr optionalen Substituenten substituiert sein kann;

Heterocycloalkyl für einen gesättigten, verzweigten oder unverzweigten carbocyclischen Ring steht, der 3 bis 15 Kohlenstoffatome enthält, wobei der carbocyclische Ring durch 1 bis 3 Heteroatome ausgewählt aus -O-, -S- und -N-unterbrochen ist, wobei der Ring gegebenenfalls ein oder zwei ungesättigte Bindungen enthalten kann, die dem Ring keinen aromatischen Charakter verleihen, und wobei irgendwelche der verfügbaren substituierbaren Kohlenstoffund Stickstoffatome in dem Ring gegebenenfalls und unabhängig mit einem, zwei, drei oder mehr optionalen Substituenten substituiert sein können; und

"optionale Substituenten" ausgewählt sind aus Halogen, Alkyl, Aryl, Alkoxy, Amino, Alkylamino, Cyano, $-CF_3$, Dialkylamino, Hydroxy, Oxy, Phenoxy, $-OCF_3$, Heterocycloalkyl, $-SO_2NH_2$, $-NHSO_2R^{10}$, $-SO_2NHR^{10}$, $SO_2R^{10}$, $-SOR^{10}$, $-SR^{10}$, $-NHSO_2$, $-NO_2$, $-CONR^{10}$, $-NCOR^{10}$ oder $-COOR^{10}$.

**2.** Verbindung nach Anspruch 1, bei der $R^2$ Halogen in der 3-Position ist und $R^3$ Halogen in der 8-Position ist.

**3.** Verbindung nach Anspruch 1, bei der $R^2$ Br in der 3-Position ist und $R^3$ Cl in der 8-Position ist.

**4.** Verbindung nach Anspruch 1, bei der $R^1$ H ist und $R^2$, $R^3$ und $R^4$ Halogen ausgewählt aus Chlor und Brom sind.

**5.** Verbindung nach Anspruch 1, bei der $R^2$ Halogen in der 3-Position ist, $R^3$ Halogen in der 8-Position ist und $R^4$ Halogen in der 10-Position ist.

**6.** Verbindung nach Anspruch 1, bei der $R^2$ Brom in der 3-Position ist, $R^3$ Chlor in der 8-Position ist und $R^4$ Brom in der 10-Position ist.

**7.** Verbindung nach einem der vorhergehenden Ansprüche, bei der $R^{40}$ H ist und $R^{42}$ 3-Pyridylmethyl ist.

**8.** Verbindung nach Anspruch 1 ausgewählt aus:

1-(3-Brom-8-chlor-6,11-dihydro-5H-benzo[5,6]cyclohepta-[1,2-b]pyridin-11-yl)-N-(3-pyridinylmethyl)2-pyrrolidincarboxamid;

1-(3,10-Dibrom-8-chlor-6,11-dihydro-5H-benzo[5,6]cyclohepta[1,2-b]pyridin-11-yl)-N-(3-pyridinylmethyl)-2-pyrrolidincarboxamid und

1-(3-Brom-8-chlor-6,11-dihydro-5H-benzo[5,6]cyclohepta-[1,2-b]pyridin-11-yl)-N-(3-pyridinylmethyl)2-azetidincarboxamid.

**9.** Verbindung nach Anspruch 8, die ausgewählt ist aus

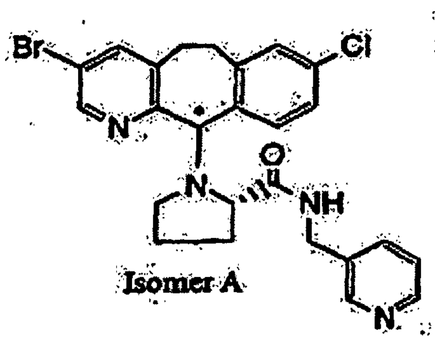

Isomer A

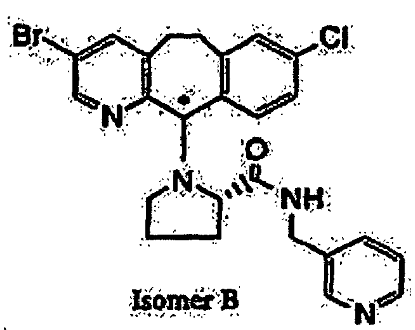

Isomer B

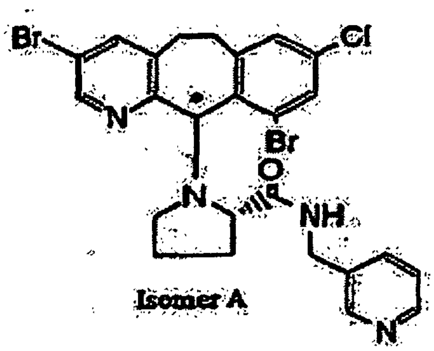

Isomer A

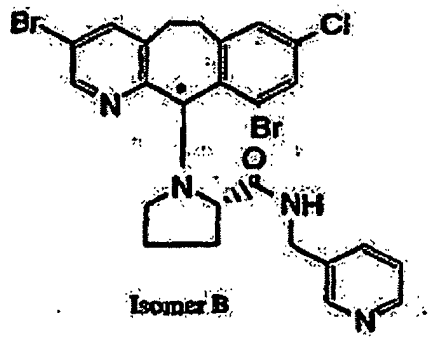

Isomer B

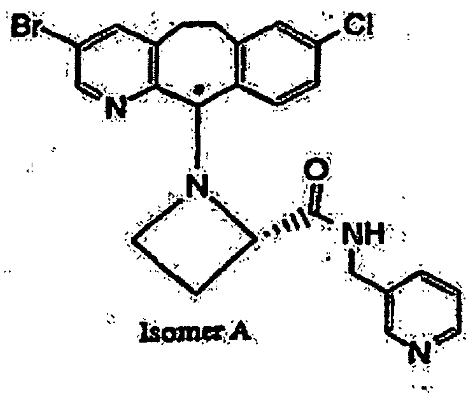

Isomer A

und

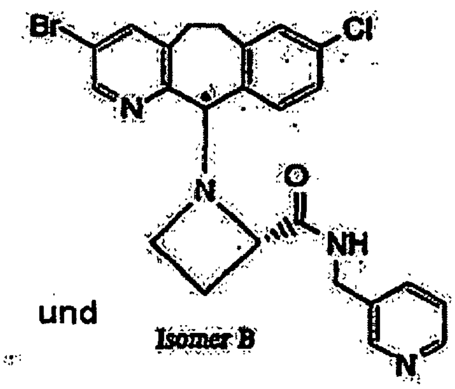

Isomer B

oder einem pharmazeutisch annehmbarem Salz davon.

**10.** Verbindung nach Anspruch 9, die wie folgt ist:

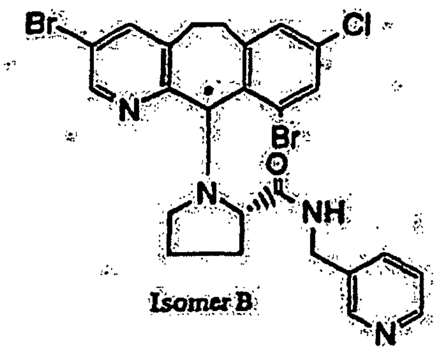

Isomer B

**11.** Pharmazeutische Zusammensetzung zum Inhibieren des abnormalen Wachstums von Zellen, die eine wirksame Menge von Verbindung nach einem der Ansprüche 1 bis 10 in Kombination mit einem pharmazeutisch annehmbaren Träger enthält.

**12.** Verbindung nach einem der Ansprüche 1 bis 10 zur Verwendung bei der Inhibierung des abnormalen Wachstums von Zellen.

**13.** Verbindung nach Anspruch 12, bei der die inhibierten Zellen Tumorzellen sind, die ein aktiviertes ras-Onkogen exprimieren.

**14.** Verbindung nach Anspruch 12, wobei die inhibierten Zellen Pankreastumorzellen, Lungenkrebszellen, myeloide

Leukämietumorzellen, Schilddrüsenfollikeltumorzellen, myelodysplastische Tumorzellen, epidermale Carcinomtumorzellen, Blasencarcinomtumorzellen oder Colontumorzellen sind.

**15.** Verbindung nach einem der Ansprüche 12 bis 14, wobei die Inhibierung des abnormalen Wachstums von Zellen durch Inhibierung von ras-Farnesylproteintransferase erfolgt.

**16.** Verbindung nach einem der Ansprüche 12 bis 15, wobei die Inhibierung, die von Tumorzellen ist, bei denen das Ras-Protein infolge von onkogener Mutation in anderen Genen als dem Ras-Gen aktiviert ist.

**Revendications**

**1.** Composé de formule :

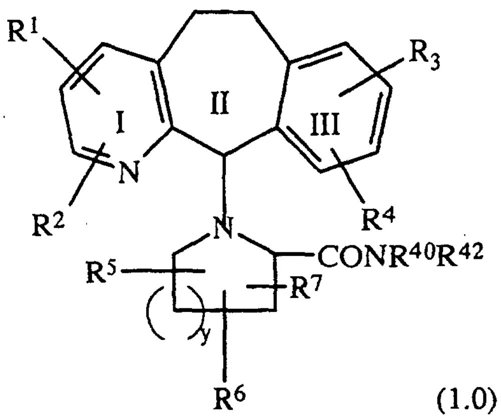

(1.0)

dans laquelle :

$R^1$ et $R^4$ représentent des atomes d'hydrogène et $R^2$ et $R^3$ représentent des atomes d'halogène choisis parmi les atomes de chlore et de brome, ou bien $R^1$ représente un atome d'hydrogène et $R^2$, $R^3$ et $R^4$ représentent des atomes d'halogène pris parmi les atomes de chlore et de brome,
$R^5$ et $R^6$ (lorsque y est égal à 0), ou bien $R^5$, $R^6$ et $R^7$ (lorsque y est égal à 1), représentent chacun indépendamment H, $-CF_3$, $-COR^{10}$, ou un groupe alkyle ou aryle, éventuellement substitué par $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$ ou $OPO_3R^{10}$, ou bien l'un de $R^5$, $R^6$ et $R^7$ peut être combiné avec $R^{40}$ défini plus loin pour former un groupe $-(CH_2)_r-$ dans lequel r représente un nombre de 1 à 4, ce groupe pouvant être substitué par un groupe alkyle en $C_1$ à $C_6$, alcoxy en $C_1$ à $C_6$, $-CF_3$ ou aryle, ou bien $R^5$ est combiné avec $R^6$ ou $R^7$ pour représenter =O ou =S,
chaque $R^{10}$ représente indépendamment H ou un groupe alkyle, cycloalkyle, cycloalkylalkyle, hétéroaryle, aryle, aralkyle ou $-NR^{40}R^{42}$,
$R^{11}$ représente un groupe alkyle ou aryle,
$R^{40}$ et $R^{42}$ représentent chacun indépendamment H, ou un groupe aryle, alkyle, aralkyle, hétéroaryle, hétéroarylalkyle, hétérocycloalkyle, hétérocycloalkylalkyle, hétéroalkyle, cycloalkyle, cycloalkylalkyle, alcényle ou alcynyle,
y est égal à 0 ou 1,
alkyle (y compris la partie alkyle des groupes alcoxy, alkylamino et dialkylamino) désigne une chaîne carbonée droite ou ramifiée, contenant 1 à 20 atomes de carbone, ledit groupe alkyle pouvant être éventuellement et indépendamment substitué par un, deux, trois ou plus de trois substituants facultatifs,
alcényle désigne une chaîne carbonée droite ou ramifiée, ayant au moins une double liaison carbone-carbone et contenant 2 à 12 atomes de carbone, ledit groupe alcényle pouvant être éventuellement et indépendamment substitué par un, deux, trois ou plus de trois substituants facultatifs,
alcynyle désigne une chaîne carbonée droite ou ramifiée, ayant au moins une triple liaison carbone-carbone et contenant 2 à 12 atomes de carbone, ledit groupe alcynyle pouvant être éventuellement et indépendamment substitué par un, deux, trois ou plus de trois substituants facultatifs,
aryle (y compris la partie aryle des groupes aralkyle) désigne un groupe carbocyclique contenant 6 à 15 atomes

de carbone et ayant au moins un noyau aromatique, ledit groupe aryle pouvant éventuellement être condensé avec des cycles aryle, cycloalkyle, hétéroaryle ou hétérocycloalkyle, et n'importe lequel des atomes de carbone et d'azote substituables disponibles dudit groupe aryle et/ou desdits noyaux condensés pouvant être éventuellement et indépendamment substitué par un ou plusieurs substituants facultatifs,
aralkyle désigne un groupe alkyle tel que défini précédemment, dont un ou plusieurs atomes d'hydrogène a (ont) été remplacé(s) par un ou plusieurs groupe aryle, ledit groupe aralkyle pouvant être éventuellement et indépendamment substitué par un, deux, trois ou plus de trois substituants facultatifs,
cycloalkyle désigne un cycle carbocyclique saturé, ramifié ou non-ramifié, ayant 3 à 20 atomes de carbone, ledit groupe cycloalkyle pouvant être éventuellement et indépendamment substitué par un, deux, trois ou plus de trois substituants facultatifs,
cycloalkylalkyle désigne un groupe alkyle tel que défini précédemment, dont un ou plusieurs atomes d'hydrogène a (ont) été remplacé(s) par un ou plusieurs groupes cycloalkyle, ledit groupe cycloalkylalkyle pouvant être éventuellement et indépendamment substitué par un, deux, trois ou plus de trois substituants facultatifs,
hétéroaryle désigne un groupe cyclique présentant au moins un hétéroatome choisi parmi O, S et N, qui interrompt une structure carbocyclique, et ayant un nombre suffisant d'électrons pi délocalisés pour avoir le caractère aromatique, ledit groupe hétérocyclique aromatique contenant 2 à 14 atomes de carbone, et ledit groupe hétéroaryle pouvant éventuellement être condensé avec un ou plusieurs cycles aryle, cycloalkyle, hétéroaryle ou hétérocycloalkyle, l'un quelconque des atomes de carbone et d'azote substituables disponibles dudit groupe hétéroaryle et/ou desdits noyaux condensés pouvant être éventuellement et indépendamment substitué par un ou plusieurs substituants facultatifs,
hétéroarylalkyle désigne un groupe alkyle tel que défini précédemment, dont un ou plusieurs atomes d'hydrogène a (ont) été remplacé(s) par un ou plusieurs groupes hétéroaryle, ledit groupe hétéroarylalkyle pouvant être éventuellement et indépendamment substitué par un, deux, trois ou plus de trois substituants facultatifs,
hétérocycloalkyle désigne un cycle carbocyclique saturé, ramifié ou non-ramifié, contenant 3 à 15 atomes de carbone, qui est interrompu par 1 à 3 hétéroatomes choisis parmi O, S et N, ledit cycle pouvant présenter éventuellement une ou plusieurs liaisons insaturées qui ne communiquent pas au cycle un caractère aromatique, et n'importe lequel des atomes de carbone et d'azote substituables disponibles du cycle pouvant être éventuellement et indépendamment substitué par un ou plusieurs substituants facultatifs, et
« substituants facultatifs » désigne des substituants choisis parmi les atomes d'halogène et les groupes alkyle, aryle, alcoxy, amino, alkylamino, cyano, $-CF_3$, dialkylamino, hydroxyle, oxy, phénoxy, $-OCF_3$, hétérocycloalkyle, $-SO_2NH_2$, $-NHSO_2R^{10}$, $-SO_2NHR^{10}$, $SO_2R^{10}$, $-SOR^{10}$, $-SR^{10}$, $-SR^{10}$, $-NHSO_2$, $-NO_2$, $-CONR^{10}$, $-NCOR^{10}$ et $-COOR^{10}$,

ou un sel ou produit de solvatation pharmaceutiquement acceptable d'un tel composé.

**2.** Composé selon la revendication 1, pour lequel $R^2$ représente un atome d'halogène en position 3 et $R^3$ représente un atome d'halogène en position 8.

**3.** Composé selon la revendication 1, pour lequel $R^2$ représente un atome de brome en position 3 et $R^3$ représente un atome de chlore en position 8.

**4.** Composé selon la revendication 1, pour lequel R' représente un atome d'hydrogène, et $R^2$, $R^3$ et $R^4$ représentent des atomes d'halogène choisis parmi les atome de chlore et de brome.

**5.** Composé selon la revendication 1, pour lequel $R^2$ représente un atome d'halogène en position 3, $R^3$ représente un atome d'halogène en position 8, et $R^4$ représente un atome d'halogène en position 10.

**6.** Composé selon la revendication 1, pour lequel $R^2$ représente un atome de brome en position 3, $R^3$ représente un atome de chlore en position 8, et $R^4$ représente un atome de brome en position 10.

**7.** Composé selon l'une quelconque des revendications précédentes, pour lequel $R^{40}$ représente un atome d'hydrogène, et $R^{42}$ représente un groupe 3-pyridylméthyle.

**8.** Composé selon la revendication 1, qui est choisi parmi les composés suivants :

le 1-(3-bromo-8-chloro-6,11-dihydro-5H-benzo[5,6]cyclohepta[1,2-b]pyridin-13-yl)-N-(3-pyridinylméthyl)-2-pyrrolidine carboxamide,
le 1-(3,10-dibromo-8-chloro-6,11-dihydro-5H-benzo[5,6]cyclohepta[1,2-b]pyridin-11-yl)-N-(3-pyridinylméthyl)-2-pyrrolidine carboxamide, et
le 1-(3-bromo-8-chloro-6,11-dihydro-5H-benzo[5,6]cyclohepta[1,2-b]pyridin-11-yl)-N-(3-pyridinylméthyl)-2-azétidine carboxamide.

**9.** Composé selon la revendication 8, qui est choisi parmi les composés suivants :

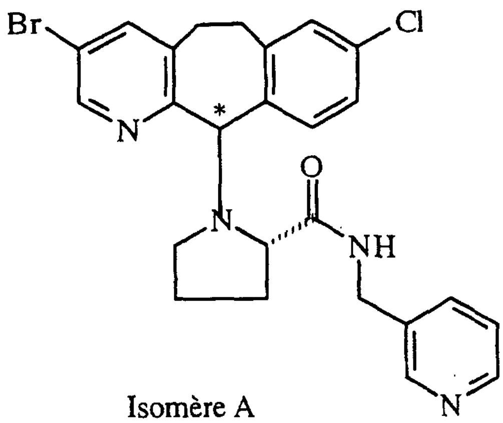

Isomère A

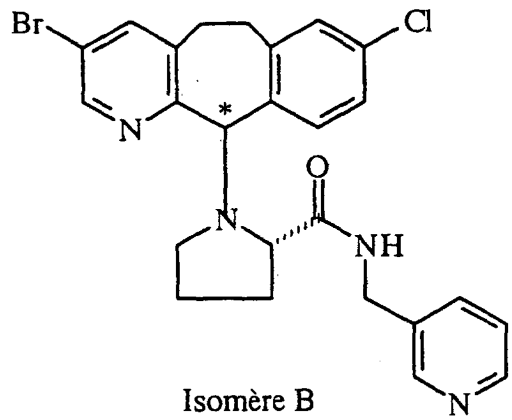

Isomère B

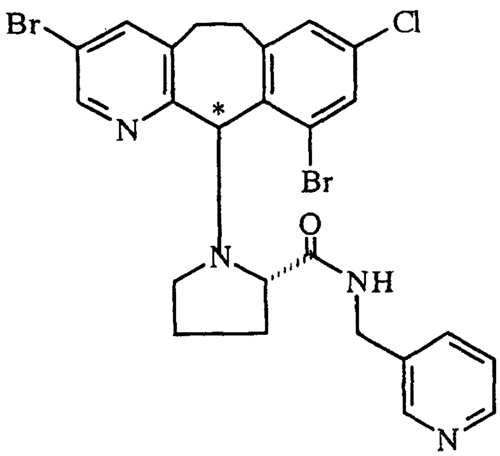

Isomère A

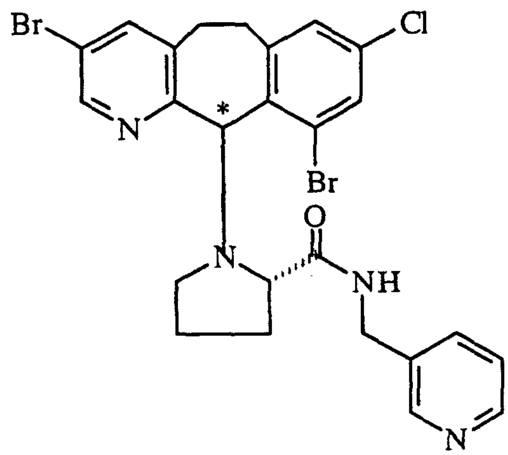

Isomère B

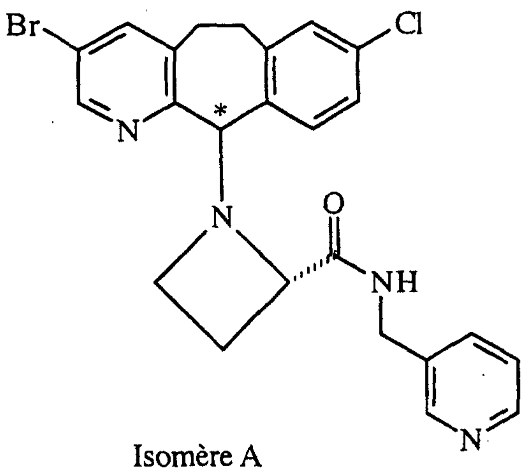

Isomère A

et

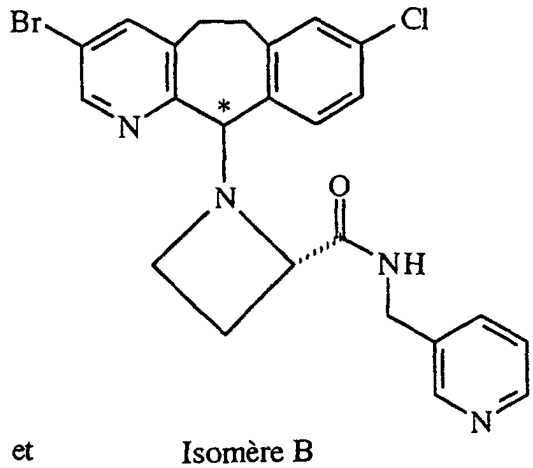

Isomère B

ou un sel pharmaceutiquement acceptable d'un tel composé.

**10.** Composé selon la revendication 9, qui est :

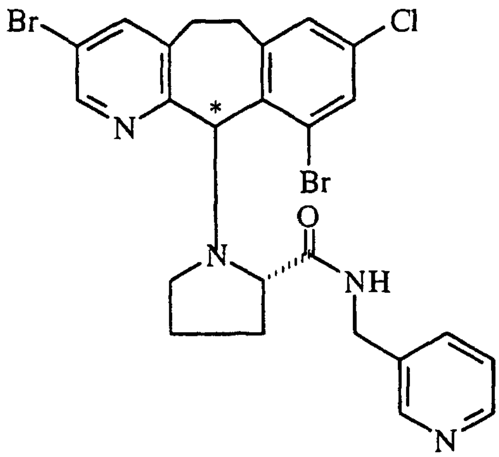

Isomère B

**11.** Composition pharmaceutique pour inhiber la croissance anormale de cellules, qui comprend une quantité efficace d'un composé selon l'une quelconque des revendications 1 à 10, en combinaison avec un support pharmaceutiquement acceptable.

**12.** Composé selon l'une quelconque des revendications 1 à 10, qui est destiné à être utilisé pour l'inhibition de la croissance anormale de cellules.

**13.** Composé selon la revendication 12, pour lequel les cellules inhibées sont des cellules tumorales exprimant un oncogène Ras activé.

**14.** Composé selon la revendication 12, pour lequel les cellules inhibées sont des cellules de tumeur pancréatique, des cellules de cancer du poumon, des cellules de tumeur de leucémie myéloïde, des cellules de tumeur folliculaire de la thyroïde, des cellules de tumeur myélodysplasique, des cellules de tumeur de carcinome de l'épiderme, des cellules de tumeur de carcinome de la vessie ou des cellules de tumeur du colon.

**15.** Composé selon l'une quelconque des revendications 12 à 14, pour lequel l'inhibition de la croissance anormale des cellules s'effectue par inhibition de la Ras farnésyl protéine transférase.

**16.** Composé selon l'une quelconque des revendications 12 à 15, pour lequel l'inhibition est celle de cellules tumorales dans lesquelles la protéine Ras est activée par suite d'une mutation oncogène dans des gènes autres que le gène Ras.